# EUROPEAN PATENT SPECIFICATION

(11) **EP 2 316 926 B1**
(45) Date of publication and mention of the grant of the patent: **13.04.2016**
(21) Application number: 09174805.3
(22) Date of filing: 02.11.2009
(51) Int. Cl.: C12N 9/02

(54) **Enantioselective production of alpha-hydroxy carbonyl compounds**
Enantioselektive Produktion von alpha-Hydroxycarbonylverbindungen
Production énantiosélective de composés alpha-hydroxy carbonyle

(43) Date of publication of application: 04.05.2011
(73) Proprietor: Rheinische Friedrich-Wilhelms-Universität Bonn, 53113 Bonn (DE)
(72) Inventor: Deppenmeier, Uwe, 53747 Lohmar (DE); Schweiger, Paul, 53115 Bonn (DE); Gross, Harald, 53757 Sankt Augustin (DE)
(74) Representative: von Kreisler Selting Werner - Partnerschaft von Patentanwälten und Rechtsanwälten mbB

(56) References cited:
- EP-A- 2 119 770
- WO-A2-2007/044043
- "Product name: Alpha-Ketocarbonyl-Reductase" Lifescienceslink.org 2 July 2009 (2009-07-02), XP002580371 Retrieved from the Internet: URL:http://www.lifescienceslink.org/techno logies-offered/to/alpha-ketocarbonyl-reduc tase-commonly-used-as-reduction-agents-how ever-the-regeneration-of-these> [retrieved on 2010-04-26]
- CHEN CHING-NEN ET AL: "Associating protein activities with their genes: rapid identification of a gene encoding a methylglyoxal reductase in the yeast Saccharomyces cerevisiae" YEAST, JOHN WILEY & SONS LTD, GB, vol. 20, no. 6, 30 April 2003 (2003-04-30) , pages 545-554, XP002487486 ISSN: 0749-503X
- PRUST CHRISTINA ET AL: "Complete genome sequence of the acetic acid bacterium Gluconobacter oxydans" NATURE BIOTECHNOLOGY, NATURE PUBLISHING GROUP, NEW YORK, NY, US, vol. 23, no. 2, 23 January 2005 (2005-01-23), pages 195-200, XP002377638 ISSN: 1087-0156 & DATABASE UniProt [Online] 1 March 2005 (2005-03-01), "SubName: Full=Putative 2,5-diketo-D-gluconic acid reductase; EC=<A HREF="http://srs.ebi.ac.uk/srsbin/cgi-bin/ wgetz?[enzyme-ECNumber:1.1.1.*]+-e">1.1.1. -</A>;" retrieved from EBI accession no. UNIPROT:Q5FT75 Database accession no. Q5FT75 & DATABASE UniProt [Online] 1 March 2005 (2005-03-01), "SubName: Full=Putative oxidoreductase; EC=<A HREF="http://srs.ebi.ac.uk/srsbin/cgi-bin/ wgetz?[enzyme-ECNumber:1.1.1.*]+-e">1.1.1. -</A>;" retrieved from EBI accession no. UNIPROT:Q5FQJ0 Database accession no. Q5FQJ0
- SCHWEIGER PAUL ET AL: "Overproduction and characterization of two distinct aldehyde-oxidizing enzymes from Gluconobacter oxydans 621H" JOURNAL OF MOLECULAR MICROBIOLOGY AND BIOTECHNOLOGY, HORIZON SCIENTIFIC PRESS, WYMONDHAM, GB LNKD- DOI:10.1159/000103606, vol. 13, no. 1-3, 1 January 2007 (2007-01-01), pages 147-155, XP009102850 ISSN: 1464-1801
- JOHANSON T ET AL: "Strain engineering for stereoselective bioreduction of dicarbonyl compounds by yeast reductases" FEMS YEAST RESEARCH, ELSEVIER SCIENCE, TOKYO, NL, vol. 5, no. 6-7, 1 April 2005 (2005-04-01) , pages 513-525, XP004789302 ISSN: 1567-1356

### SEQUENCE LISTING

<110> Rheinische Friedrich-Wilhelms-Universitaet Bonn
<120> Enantioselective Production of alpha-Hydroxy Carbonyl Compounds
<130> 093497ep
<160> 13
<170> PatentIn version 3.3
<210> 1
   <211> 840
   <212> DNA
   <213> Gluconobacter oxydans
<220>
   <221> CDS
   <222> (1)..(837)
<400> 1
   atg tcg tca cag gtt cca tcc gcc gag gcc cag acc gtg att tcc ttt       48
   Met Ser Ser Gln Val Pro Ser Ala Glu Ala Gln Thr Val Ile Ser Phe
   1               5                   10                  15
   cat gac ggt cac acc atg ccc cag atc ggg ctg ggc gtg tgg gaa acg       96
   His Asp Gly His Thr Met Pro Gln Ile Gly Leu Gly Val Trp Glu Thr
               20                  25                  30
   ccg ccg gat gag acg gcc gag gtc gtg aag gaa gcc gtg aag ctc ggt      144
   Pro Pro Asp Glu Thr Ala Glu Val Val Lys Glu Ala Val Lys Leu Gly
           35                  40                  45
   tac cgg tct gtc gat acg gcg cgt ctg tac aag aac gag gaa ggt gtc      192
   Tyr Arg Ser Val Asp Thr Ala Arg Leu Tyr Lys Asn Glu Glu Gly Val
       50                  55                  60
   ggc aaa ggt ctg gaa gac cat ccg gaa atc ttc ctg acg acc aag ctc      240
   Gly Lys Gly Leu Glu Asp His Pro Glu Ile Phe Leu Thr Thr Lys Leu
   65                  70                  75                  80
   tgg aat gac gag cag ggc tat gac agc acc ctg cgg gcg tat gaa gaa      288
   Trp Asn Asp Glu Gln Gly Tyr Asp Ser Thr Leu Arg Ala Tyr Glu Glu
                   85                  90                  95
   agc gcg cgc ctg ctg cgt cgt ccg gtg ctg gac ctg tat ctg atc cac      336
   Ser Ala Arg Leu Leu Arg Arg Pro Val Leu Asp Leu Tyr Leu Ile His
               100                 105                 110
   tgg ccg atg ccg gct cag ggg cag tat gtc gag acg tgg aag gca ctc      384
   Trp Pro Met Pro Ala Gln Gly Gln Tyr Val Glu Thr Trp Lys Ala Leu
           115                 120                 125
   gtc gag ctg aag aaa tcc ggt cgt gtg aag tcc atc ggc gtg tcc aat      432
   Val Glu Leu Lys Lys Ser Gly Arg Val Lys Ser Ile Gly Val Ser Asn
       130                 135                 140
   ttc gag tcg gag cat ctg gag cgg atc atg gat gcc acg ggt gtc gtg      480
   Phe Glu Ser Glu His Leu Glu Arg Ile Met Asp Ala Thr Gly Val Val
   145                 150                 155                 160
   ccg gtc gtc aac cag atc gag ctg cat ccc gat ttc cag cag cgc gcc      528
   Pro Val Val Asn Gln Ile Glu Leu His Pro Asp Phe Gln Gln Arg Ala
                   165                 170                 175
   ctg cgg gaa ttc cac gag aag cac aac atc cgc acc gag tcc tgg cgc      576
   Leu Arg Glu Phe His Glu Lys His Asn Ile Arg Thr Glu Ser Trp Arg
               180                 185                 190
   ccg ctg ggc aag ggg cgc gtc ctg agc gat gag cgg atc ggg aag atc      624
   Pro Leu Gly Lys Gly Arg Val Leu Ser Asp Glu Arg Ile Gly Lys Ile
           195                 200                 205
   gct gaa aag cac agc cgg act ccg gcg cag gtc gtg atc cgc tgg cat      672
   Ala Glu Lys His Ser Arg Thr Pro Ala Gln Val Val Ile Arg Trp His
       210                 215                 220
   ctt cag aat gga ctg atc gtc att ccg aaa tcg gtc aat ccc aag cgt      720
   Leu Gln Asn Gly Leu Ile Val Ile Pro Lys Ser Val Asn Pro Lys Arg
   225                 230                 235                 240
   ctg gct gaa aat ctg gat gtg ttc ggc ttc gtg ctg gat gcg gat gac      768
   Leu Ala Glu Asn Leu Asp Val Phe Gly Phe Val Leu Asp Ala Asp Asp
                   245                 250                 255
   atg cag gcc atc gaa cag atg gac cgc aag gat ggc cgg atg ggc gct      816
   Met Gln Ala Ile Glu Gln Met Asp Arg Lys Asp Gly Arg Met Gly Ala
               260                 265                 270
   gat ccg aat acg gcg aaa ttc tga                                      840
   Asp Pro Asn Thr Ala Lys Phe
           275
<210> 2
   <211> 279
   <212> PRT
   <213> Gluconobacter oxydans
<400> 2
   Met Ser Ser Gln Val Pro Ser Ala Glu Ala Gln Thr Val Ile Ser Phe
   1               5                   10                  15
   His Asp Gly His Thr Met Pro Gln Ile Gly Leu Gly Val Trp Glu Thr
               20                  25                  30
   Pro Pro Asp Glu Thr Ala Glu Val Val Lys Glu Ala Val Lys Leu Gly
           35                  40                  45
   Tyr Arg Ser Val Asp Thr Ala Arg Leu Tyr Lys Asn Glu Glu Gly Val
       50                  55                  60
   Gly Lys Gly Leu Glu Asp His Pro Glu Ile Phe Leu Thr Thr Lys Leu
   65                  70                  75                  80
   Trp Asn Asp Glu Gln Gly Tyr Asp Ser Thr Leu Arg Ala Tyr Glu Glu
                   85                  90                  95
   Ser Ala Arg Leu Leu Arg Arg Pro Val Leu Asp Leu Tyr Leu Ile His
               100                 105                 110
   Trp Pro Met Pro Ala Gln Gly Gln Tyr Val Glu Thr Trp Lys Ala Leu
           115                 120                 125
   Val Glu Leu Lys Lys Ser Gly Arg Val Lys Ser Ile Gly Val Ser Asn
       130                 135                 140
   Phe Glu Ser Glu His Leu Glu Arg Ile Met Asp Ala Thr Gly Val Val
   145                 150                 155                 160
   Pro Val Val Asn Gln Ile Glu Leu His Pro Asp Phe Gln Gln Arg Ala
                   165                 170                 175
   Leu Arg Glu Phe His Glu Lys His Asn Ile Arg Thr Glu Ser Trp Arg
               180                 185                 190
   Pro Leu Gly Lys Gly Arg Val Leu Ser Asp Glu Arg Ile Gly Lys Ile
           195                 200                 205
   Ala Glu Lys His Ser Arg Thr Pro Ala Gln Val Val Ile Arg Trp His
       210                 215                 220
   Leu Gln Asn Gly Leu Ile Val Ile Pro Lys Ser Val Asn Pro Lys Arg
   225                 230                 235                 240
   Leu Ala Glu Asn Leu Asp Val Phe Gly Phe Val Leu Asp Ala Asp Asp
                   245                 250                 255
   Met Gln Ala Ile Glu Gln Met Asp Arg Lys Asp Gly Arg Met Gly Ala
               260                 265                 270
   Asp Pro Asn Thr Ala Lys Phe
           275
<210> 3
   <211> 999
   <212> DNA
   <213> Gluconobacter oxydans
<220>
   <221> CDS
   <222> (1)..(996)
<400> 3
   atg gca tcc gac acc atc cgc atc ccc ggc atc gac acg cct ctc tcc        48
   Met Ala Ser Asp Thr Ile Arg Ile Pro Gly Ile Asp Thr Pro Leu Ser
   1               5                   10                  15
   cgc gtc gcc ctc ggc acc tgg gcc atc ggc ggc tgg atg tgg ggc ggc       96
   Arg Val Ala Leu Gly Thr Trp Ala Ile Gly Gly Trp Met Trp Gly Gly
               20                  25                  30
   ccc gat gat gac aat ggc gtc cgc acc att cac gcg gcc ctc gat gaa      144
   Pro Asp Asp Asp Asn Gly Val Arg Thr Ile His Ala Ala Leu Asp Glu
           35                  40                  45
   ggc atc aac ctg atc gac acc gcc ccg gtc tac ggc ttc ggc cat tcc      192
   Gly Ile Asn Leu Ile Asp Thr Ala Pro Val Tyr Gly Phe Gly His Ser
       50                  55                  60
   gag gaa atc gtc ggc cgg gca ctc gcc gag aag ccc aac aag gcg cat      240
   Glu Glu Ile Val Gly Arg Ala Leu Ala Glu Lys Pro Asn Lys Ala His
   65                  70                  75                  80
   gtc gcc acg aaa ctc ggg ctc cac tgg gtt ggc gaa gac gaa aag aat      288
   Val Ala Thr Lys Leu Gly Leu His Trp Val Gly Glu Asp Glu Lys Asn
                   85                  90                  95
   atg aag gtc ttc cgg gac tcc cgc ccg gcc cgc atc cgc aag gaa gtc      336
   Met Lys Val Phe Arg Asp Ser Arg Pro Ala Arg Ile Arg Lys Glu Val
               100                 105                 110
   gag gac tcg ctc cgc cgt ctg cgt gtg gag acg atc gat ctc gag cag      384
   Glu Asp Ser Leu Arg Arg Leu Arg Val Glu Thr Ile Asp Leu Glu Gln
           115                 120                 125
   atc cac tgg ccc gac gac aaa acc ccg atc gac gag agc gcc cgc gag      432
   Ile His Trp Pro Asp Asp Lys Thr Pro Ile Asp Glu Ser Ala Arg Glu
       130                 135                 140
   ctt cag aaa ctc cat cag gac ggc aag atc cgt gcc ctc ggc gta agc      480
   Leu Gln Lys Leu His Gln Asp Gly Lys Ile Arg Ala Leu Gly Val Ser
   145                 150                 155                 160
   aac ttc tcg ccg gag cag atg gac atc ttc cgc gaa gtc gcc ccg ctc      528
   Asn Phe Ser Pro Glu Gln Met Asp Ile Phe Arg Glu Val Ala Pro Leu
                   165                 170                 175
   gcc acg atc cag ccc ccg ctg aac ctt ttc gaa cgc acc atc gag aag      576
   Ala Thr Ile Gln Pro Pro Leu Asn Leu Phe Glu Arg Thr Ile Glu Lys
               180                 185                 190
   gac atc ctg ccc tac gcc gaa aag cac aat gcc gtc gtt ctc gct tac      624
   Asp Ile Leu Pro Tyr Ala Glu Lys His Asn Ala Val Val Leu Ala Tyr
           195                 200                 205
   ggg gcc ctc tgc cgc ggc ctg ctg aca ggc aag atg aac cgc gat acg      672
   Gly Ala Leu Cys Arg Gly Leu Leu Thr Gly Lys Met Asn Arg Asp Thr
       210                 215                 220
   acc ttc ccg aag gat gac ctg cgt tcg aac gat ccg aag ttc cag aag      720
   Thr Phe Pro Lys Asp Asp Leu Arg Ser Asn Asp Pro Lys Phe Gln Lys
   225                 230                 235                 240
   ccg aat ttc gag aag tac ctc gca gcc atg gac gag ttc gaa aag ctt      768
   Pro Asn Phe Glu Lys Tyr Leu Ala Ala Met Asp Glu Phe Glu Lys Leu
                   245                 250                 255
   gcc gaa aag cgc ggc aag tct gtg atg gcc ttc gcg gtt cgc tgg gtt      816
   Ala Glu Lys Arg Gly Lys Ser Val Met Ala Phe Ala Val Arg Trp Val
               260                 265                 270
   ctg gat cag ggc ccg gta atc gcc ctg tgg ggt gcc cgc aag ccg ggt        864
   Leu Asp Gln Gly Pro Val Ile Ala Leu Trp Gly Ala Arg Lys Pro Gly
           275                 280                 285
   cag gtc tca ggc gta aag gac gtc ttc ggc tgg tcc ctg acc gac gaa        912
   Gln Val Ser Gly Val Lys Asp Val Phe Gly Trp Ser Leu Thr Asp Glu
       290                 295                 300
   gag aag aaa gcc gtg gac gac att ctg gct agg cat gtc ccc aat ccc        960
   Glu Lys Lys Ala Val Asp Asp Ile Leu Ala Arg His Val Pro Asn Pro
   305                 310                 315                 320
   atc gac ccg act ttc atg gcg ccc ccg gca cgg gac tga                    999
   Ile Asp Pro Thr Phe Met Ala Pro Pro Ala Arg Asp
                   325                 330
<210> 4
   <211> 332
   <212> PRT
   <213> Gluconobacter oxydans
<400> 4
   Met Ala Ser Asp Thr Ile Arg Ile Pro Gly Ile Asp Thr Pro Leu Ser
   1               5                   10                  15
   Arg Val Ala Leu Gly Thr Trp Ala Ile Gly Gly Trp Met Trp Gly Gly
               20                  25                  30
   Pro Asp Asp Asp Asn Gly Val Arg Thr Ile His Ala Ala Leu Asp Glu
           35                  40                  45
   Gly Ile Asn Leu Ile Asp Thr Ala Pro Val Tyr Gly Phe Gly His Ser
       50                  55                  60
   Glu Glu Ile Val Gly Arg Ala Leu Ala Glu Lys Pro Asn Lys Ala His
   65                  70                  75                  80
   Val Ala Thr Lys Leu Gly Leu His Trp Val Gly Glu Asp Glu Lys Asn
                   85                  90                  95
   Met Lys Val Phe Arg Asp Ser Arg Pro Ala Arg Ile Arg Lys Glu Val
               100                 105                 110
   Glu Asp Ser Leu Arg Arg Leu Arg Val Glu Thr Ile Asp Leu Glu Gln
           115                 120                 125
   Ile His Trp Pro Asp Asp Lys Thr Pro Ile Asp Glu Ser Ala Arg Glu
       130                 135                 140
   Leu Gln Lys Leu His Gln Asp Gly Lys Ile Arg Ala Leu Gly Val Ser
   145                 150                 155                 160
   Asn Phe Ser Pro Glu Gln Met Asp Ile Phe Arg Glu Val Ala Pro Leu
                   165                 170                 175
   Ala Thr Ile Gln Pro Pro Leu Asn Leu Phe Glu Arg Thr Ile Glu Lys
               180                 185                 190
   Asp Ile Leu Pro Tyr Ala Glu Lys His Asn Ala Val Val Leu Ala Tyr
           195                 200                 205
   Gly Ala Leu Cys Arg Gly Leu Leu Thr Gly Lys Met Asn Arg Asp Thr
       210                 215                 220
   Thr Phe Pro Lys Asp Asp Leu Arg Ser Asn Asp Pro Lys Phe Gln Lys
   225                 230                 235                 240
   Pro Asn Phe Glu Lys Tyr Leu Ala Ala Met Asp Glu Phe Glu Lys Leu
                   245                 250                 255
   Ala Glu Lys Arg Gly Lys Ser Val Met Ala Phe Ala Val Arg Trp Val
               260                 265                 270
   Leu Asp Gln Gly Pro Val Ile Ala Leu Trp Gly Ala Arg Lys Pro Gly
           275                 280                 285
   Gln Val Ser Gly Val Lys Asp Val Phe Gly Trp Ser Leu Thr Asp Glu
       290                 295                 300
   Glu Lys Lys Ala Val Asp Asp Ile Leu Ala Arg His Val Pro Asn Pro
   305                 310                 315                 320
   Ile Asp Pro Thr Phe Met Ala Pro Pro Ala Arg Asp
                   325                 330
<210> 5
   <211> 1392
   <212> DNA
   <213> Gluconobacter oxydans
<220>
   <221> CDS
   <222> (1)..(1389)
<400> 5
   atg gct tac gct acg atc aac cct tat acc ggt gag acg ctc aag acc        48
   Met Ala Tyr Ala Thr Ile Asn Pro Tyr Thr Gly Glu Thr Leu Lys Thr
   1               5                   10                  15
   ttc ccg gaa gca acc gac cag gaa gtc cag acc gcg ctg acc gaa gcc        96
   Phe Pro Glu Ala Thr Asp Gln Glu Val Gln Thr Ala Leu Thr Glu Ala
               20                  25                  30
   tat gat gcg ttc aag agc tgg cgt cat acg ccg ttc gcc gag cgc gca      144
   Tyr Asp Ala Phe Lys Ser Trp Arg His Thr Pro Phe Ala Glu Arg Ala
           35                  40                  45
   aag atc atg acg gca gcg gcg aac att ctt cgc cgt gat atc gac aag      192
   Lys Ile Met Thr Ala Ala Ala Asn Ile Leu Arg Arg Asp Ile Asp Lys
       50                  55                  60
   tat tcc aag ctt ctg acg ctc gag atg ggc aag atc tat gcc gaa gcc      240
   Tyr Ser Lys Leu Leu Thr Leu Glu Met Gly Lys Ile Tyr Ala Glu Ala
   65                  70                  75                  80
   aag gca gag acg atc ctg tcc gcc gag atc ttc gaa tat tat gcc aag      288
   Lys Ala Glu Thr Ile Leu Ser Ala Glu Ile Phe Glu Tyr Tyr Ala Lys
                   85                  90                  95
   aac gcc gaa gaa ctg ctg aaa gcc gag aag ctg ccg gtt gcg gat ccg      336
   Asn Ala Glu Glu Leu Leu Lys Ala Glu Lys Leu Pro Val Ala Asp Pro
               100                 105                 110
   aag gaa ggc gag gcc gtc ctg atc cac cag ccg cag ggc att gtg ttc      384
   Lys Glu Gly Glu Ala Val Leu Ile His Gln Pro Gln Gly Ile Val Phe
           115                 120                 125
   gcg atc gag ccg tgg aac ttc ccg ttc tac cag atc gcc cgc atc atc      432
   Ala Ile Glu Pro Trp Asn Phe Pro Phe Tyr Gln Ile Ala Arg Ile Ile
       130                 135                 140
   gcg ccg cag ctc tcc gcc ggt aac acg gtc att ctc aag cac gcc tcc      480
   Ala Pro Gln Leu Ser Ala Gly Asn Thr Val Ile Leu Lys His Ala Ser
   145                 150                 155                 160
   aac gtg ccg cag tgt gct gct gcg ttc gat gac ctg atg aag gaa gcc      528
   Asn Val Pro Gln Cys Ala Ala Ala Phe Asp Asp Leu Met Lys Glu Ala
                   165                 170                 175
   ggt ctg ccg aag ggt gga ttc cgc aac ctg tat gct gcc cgt cat cac      576
   Gly Leu Pro Lys Gly Gly Phe Arg Asn Leu Tyr Ala Ala Arg His His
               180                 185                 190
   acc gag acg atc ctc aag gac ccg cgc gtc tgt ggc gtg gcc ctg acg      624
   Thr Glu Thr Ile Leu Lys Asp Pro Arg Val Cys Gly Val Ala Leu Thr
           195                 200                 205
   ggt tcg gaa ggt gct ggc gct gtt gtg gcc ggg att gcg gca cag gcg      672
   Gly Ser Glu Gly Ala Gly Ala Val Val Ala Gly Ile Ala Ala Gln Ala
       210                 215                 220
   ctg aag aag tcc acg atg gag ctc ggg ggt tcc gat gcg ttc ctc gtt      720
   Leu Lys Lys Ser Thr Met Glu Leu Gly Gly Ser Asp Ala Phe Leu Val
   225                 230                 235                 240
   ctg gac gat gcg gat ctg gaa aag acc gcg aag tgg gct gct ttc ggc      768
   Leu Asp Asp Ala Asp Leu Glu Lys Thr Ala Lys Trp Ala Ala Phe Gly
                   245                 250                 255
   cgt cac tgg aat gcc ggt cag gtc tgc gtg tcc gca aag cgt ctg atc      816
   Arg His Trp Asn Ala Gly Gln Val Cys Val Ser Ala Lys Arg Leu Ile
               260                 265                 270
   gtg gcg gat gcc gtg tat gat cgc ttc gtc gag ctg tac aag gcc gaa      864
   Val Ala Asp Ala Val Tyr Asp Arg Phe Val Glu Leu Tyr Lys Ala Glu
           275                 280                 285
   gtc gcc aag ctg atc ccg ggt gat ccg atg gac ccg aag acc acg ctg      912
   Val Ala Lys Leu Ile Pro Gly Asp Pro Met Asp Pro Lys Thr Thr Leu
       290                 295                 300
   gct ccg ctg tcc tcg cag aag gct gct gat gat ctg aag gct cag gtc      960
   Ala Pro Leu Ser Ser Gln Lys Ala Ala Asp Asp Leu Lys Ala Gln Val
   305                 310                 315                 320
   gag gaa gcc aag aag cac ggt gcc gtc gtc gag gcc att ggt ccg tcc     1008
   Glu Glu Ala Lys Lys His Gly Ala Val Val Glu Ala Ile Gly Pro Ser
                   325                 330                 335
   gta ccg gag aag ggt gcc ttc ttc cag ccg ctg ctg atg acc aac ctg     1056
   Val Pro Glu Lys Gly Ala Phe Phe Gln Pro Leu Leu Met Thr Asn Leu
               340                 345                 350
   cac gag aac aat gaa gcc cgt ctg tgg gag ttc ttt ggt ccg gtg acg     1104
   His Glu Asn Asn Glu Ala Arg Leu Trp Glu Phe Phe Gly Pro Val Thr
           355                 360                 365
   cag ctt tat cgt gcc aag gac gat gcg gat gcc atc cgt atc gcc aat     1152
   Gln Leu Tyr Arg Ala Lys Asp Asp Ala Asp Ala Ile Arg Ile Ala Asn
       370                 375                 380
   gac tcg ccg ttc ggt ctg ggt ggt tcg gtc ttc acg aag gac acc cag     1200
   Asp Ser Pro Phe Gly Leu Gly Gly Ser Val Phe Thr Lys Asp Thr Gln
   385                 390                 395                 400
   cgt ggc gtg aag gtt gcc gag cag atc tat acg ggc atg gtc tat atc     1248
   Arg Gly Val Lys Val Ala Glu Gln Ile Tyr Thr Gly Met Val Tyr Ile
                   405                 410                 415
   aac cat ccg acg atg gtg aag gct gat ctg ccg ttc ggt ggc gtg gcc     1296
   Asn His Pro Thr Met Val Lys Ala Asp Leu Pro Phe Gly Gly Val Ala
               420                 425                 430
   cgt tcg ggt tat ggc cgt gag ctg atc ggt ctg ggt atc aag gaa ttc     1344
   Arg Ser Gly Tyr Gly Arg Glu Leu Ile Gly Leu Gly Ile Lys Glu Phe
           435                 440                 445
   gtc aat cac aag ctg atc gac gtt gtt gat atc gac gcg ccg ttc tga     1392
   Val Asn His Lys Leu Ile Asp Val Val Asp Ile Asp Ala Pro Phe
       450                 455                 460
<210> 6
   <211> 463
   <212> PRT
   <213> Gluconobacter oxydans
<400> 6
   Met Ala Tyr Ala Thr Ile Asn Pro Tyr Thr Gly Glu Thr Leu Lys Thr
   1               5                   10                  15
   Phe Pro Glu Ala Thr Asp Gln Glu Val Gln Thr Ala Leu Thr Glu Ala
               20                  25                  30
   Tyr Asp Ala Phe Lys Ser Trp Arg His Thr Pro Phe Ala Glu Arg Ala
           35                  40                  45
   Lys Ile Met Thr Ala Ala Ala Asn Ile Leu Arg Arg Asp Ile Asp Lys
       50                  55                  60
   Tyr Ser Lys Leu Leu Thr Leu Glu Met Gly Lys Ile Tyr Ala Glu Ala
   65                  70                  75                  80
   Lys Ala Glu Thr Ile Leu Ser Ala Glu Ile Phe Glu Tyr Tyr Ala Lys
                   85                  90                  95
   Asn Ala Glu Glu Leu Leu Lys Ala Glu Lys Leu Pro Val Ala Asp Pro
               100                 105                 110
   Lys Glu Gly Glu Ala Val Leu Ile His Gln Pro Gln Gly Ile Val Phe
           115                 120                 125
   Ala Ile Glu Pro Trp Asn Phe Pro Phe Tyr Gln Ile Ala Arg Ile Ile
       130                 135                 140
   Ala Pro Gln Leu Ser Ala Gly Asn Thr Val Ile Leu Lys His Ala Ser
   145                 150                 155                 160
   Asn Val Pro Gln Cys Ala Ala Ala Phe Asp Asp Leu Met Lys Glu Ala
                   165                 170                 175
   Gly Leu Pro Lys Gly Gly Phe Arg Asn Leu Tyr Ala Ala Arg His His
               180                 185                 190
   Thr Glu Thr Ile Leu Lys Asp Pro Arg Val Cys Gly Val Ala Leu Thr
           195                 200                 205
   Gly Ser Glu Gly Ala Gly Ala Val Val Ala Gly Ile Ala Ala Gln Ala
       210                 215                 220
   Leu Lys Lys Ser Thr Met Glu Leu Gly Gly Ser Asp Ala Phe Leu Val
   225                 230                 235                 240
   Leu Asp Asp Ala Asp Leu Glu Lys Thr Ala Lys Trp Ala Ala Phe Gly
                   245                 250                 255
   Arg His Trp Asn Ala Gly Gln Val Cys Val Ser Ala Lys Arg Leu Ile
               260                 265                 270
   Val Ala Asp Ala Val Tyr Asp Arg Phe Val Glu Leu Tyr Lys Ala Glu
           275                 280                 285
   Val Ala Lys Leu Ile Pro Gly Asp Pro Met Asp Pro Lys Thr Thr Leu
       290                 295                 300
   Ala Pro Leu Ser Ser Gln Lys Ala Ala Asp Asp Leu Lys Ala Gln Val
   305                 310                 315                 320
   Glu Glu Ala Lys Lys His Gly Ala Val Val Glu Ala Ile Gly Pro Ser
                   325                 330                 335
   Val Pro Glu Lys Gly Ala Phe Phe Gln Pro Leu Leu Met Thr Asn Leu
               340                 345                 350
   His Glu Asn Asn Glu Ala Arg Leu Trp Glu Phe Phe Gly Pro Val Thr
           355                 360                 365
   Gln Leu Tyr Arg Ala Lys Asp Asp Ala Asp Ala Ile Arg Ile Ala Asn
       370                 375                 380
   Asp Ser Pro Phe Gly Leu Gly Gly Ser Val Phe Thr Lys Asp Thr Gln
   385                 390                 395                 400
   Arg Gly Val Lys Val Ala Glu Gln Ile Tyr Thr Gly Met Val Tyr Ile
                   405                 410                 415
   Asn His Pro Thr Met Val Lys Ala Asp Leu Pro Phe Gly Gly Val Ala
               420                 425                 430
   Arg Ser Gly Tyr Gly Arg Glu Leu Ile Gly Leu Gly Ile Lys Glu Phe
           435                 440                 445
   Val Asn His Lys Leu Ile Asp Val Val Asp Ile Asp Ala Pro Phe
       450                 455                 460
<210> 7
   <211> 39
   <212> DNA
   <213> Artificial
<220>
   <223> primer
<400> 7
   atggtaggtc tcagcgcctc gtcacaggtt ccatccgcc 39
<210> 8
   <211> 41
   <212> DNA
   <213> Artificial
<220>
   <223> primer
<400> 8
   atggtaggtc tcatatcaga atttcgccgt attcggatca g 41
<210> 9
   <211> 39
   <212> DNA
   <213> Artificial
<220>
   <223> primer
<400> 9
   atggtacgtc tcagcgccgc atccgacacc atccgcatc 39
<210> 10
   <211> 36
   <212> DNA
   <213> Artificial
<220>
   <223> primer
<400> 10
   atggtacgtc tcatatcagt cccgtgccgg gggcgc 36
<210> 11
   <211> 41
   <212> DNA
   <213> Artificial
<220>
   <223> primer
<400> 11
   atggtaggtc tcagcgccgc ttacgctacg atcaaccctt a 41
<210> 12
   <211> 40
   <212> DNA
   <213> Artificial
<220>
   <223> primer
<400> 12
   atggtaggtc tcatatcaga acggcgcgtc gatatcaaca 40
<210> 13
   <211> 3239
   <212> DNA
   <213> Artificial
<220>
   <223> vector pASK-IBA5
<400> 13
   ccatcgaatg gccagatgat taattcctaa tttttgttga cactctatca ttgatagagt      60
   tattttacca ctccctatca gtgatagaga aaagtgaaat gaatagttcg acaaaaatct     120
   agataacgag ggcaaaaaat ggctagctgg agccacccgc agttcgaaaa aggcgccgag     180
   accgcggtcc cgaattcgag ctcggtaccc ggggatccct cgaggtcgac ctgcaggggg     240
   accatggtct ctgatatcta actaagcttg acctgtgaag tgaaaaatgg cgcacattgt     300
   gcgacatttt ttttgtctgc cgtttaccgc tactgcgtca cggatctcca cgcgccctgt     360
   agcggcgcat taagcgcggc gggtgtggtg gttacgcgca gcgtgaccgc tacacttgcc     420
   agcgccctag cgcccgctcc tttcgctttc ttcccttcct ttctcgccac gttcgccggc     480
   tttccccgtc aagctctaaa tcgggggctc cctttagggt tccgatttag tgctttacgg     540
   cacctcgacc ccaaaaaact tgattagggt gatggttcac gtagtgggcc atcgccctga     600
   tagacggttt ttcgcccttt gacgttggag tccacgttct ttaatagtgg actcttgttc     660
   caaactggaa caacactcaa ccctatctcg gtctattctt ttgatttata agggattttg     720
   ccgatttcgg cctattggtt aaaaaatgag ctgatttaac aaaaatttaa cgcgaatttt     780
   aacaaaatat taacgcttac aatttcaggt ggcacttttc ggggaaatgt gcgcggaacc     840
   cctatttgtt tatttttcta aatacattca aatatgtatc cgctcatgag acaataaccc     900
   tgataaatgc ttcaataata ttgaaaaagg aagagtatga gtattcaaca tttccgtgtc     960
   gcccttattc ccttttttgc ggcattttgc cttcctgttt ttgctcaccc agaaacgctg    1020
   gtgaaagtaa aagatgctga agatcagttg ggtgcacgag tgggttacat cgaactggat    1080
   ctcaacagcg gtaagatcct tgagagtttt cgccccgaag aacgttttcc aatgatgagc    1140
   acttttaaag ttctgctatg tggcgcggta ttatcccgta ttgacgccgg gcaagagcaa    1200
   ctcggtcgcc gcatacacta ttctcagaat gacttggttg agtactcacc agtcacagaa    1260
   aagcatctta cggatggcat gacagtaaga gaattatgca gtgctgccat aaccatgagt    1320
   gataacactg cggccaactt acttctgaca acgatcggag gaccgaagga gctaaccgct    1380
   tttttgcaca acatggggga tcatgtaact cgccttgatc gttgggaacc ggagctgaat    1440
   gaagccatac caaacgacga gcgtgacacc acgatgcctg tagcaatggc aacaacgttg    1500
   cgcaaactat taactggcga actacttact ctagcttccc ggcaacaatt gatagactgg    1560
   atggaggcgg ataaagttgc aggaccactt ctgcgctcgg cccttccggc tggctggttt    1620
   attgctgata aatctggagc cggtgagcgt ggctctcgcg gtatcattgc agcactgggg    1680
   ccagatggta agccctcccg tatcgtagtt atctacacga cggggagtca ggcaactatg    1740
   gatgaacgaa atagacagat cgctgagata ggtgcctcac tgattaagca ttggtaggaa    1800
   ttaatgatgt ctcgtttaga taaaagtaaa gtgattaaca gcgcattaga gctgcttaat    1860
   gaggtcggaa tcgaaggttt aacaacccgt aaactcgccc agaagctagg tgtagagcag   1920
   cctacattgt attggcatgt aaaaaataag cgggctttgc tcgacgcctt agccattgag   1980
   atgttagata ggcaccatac tcacttttgc cctttagaag gggaaagctg gcaagatttt   2040
   ttacgtaata acgctaaaag ttttagatgt gctttactaa gtcatcgcga tggagcaaaa   2100
   gtacatttag gtacacggcc tacagaaaaa cagtatgaaa ctctcgaaaa tcaattagcc   2160
   tttttatgcc aacaaggttt ttcactagag aatgcattat atgcactcag cgcagtgggg   2220
   cattttactt taggttgcgt attggaagat caagagcatc aagtcgctaa agaagaaagg   2280
   gaaacaccta ctactgatag tatgccgcca ttattacgac aagctatcga attatttgat   2340
   caccaaggtg cagagccagc cttcttattc ggccttgaat tgatcatatg cggattagaa   2400
   aaacaactta aatgtgaaag tgggtcttaa aagcagcata acctttttcc gtgatggtaa   2460
   cttcactagt ttaaaaggat ctaggtgaag atcctttttg ataatctcat gaccaaaatc   2520
   ccttaacgtg agttttcgtt ccactgagcg tcagaccccg tagaaaagat caaaggatct   2580
   tcttgagatc cttttttctt gcgcgtaatc tgctgcttgc aaacaaaaaa accaccgcta   2640
   ccagcggtgg tttgtttgcc ggatcaagag ctaccaactc tttttccgaa ggtaactggc   2700
   ttcagcagag cgcagatacc aaatactgtc cttctagtgt agccgtagtt aggccaccac   2760
   ttcaagaact ctgtagcacc gcctacatac ctcgctctgc taatcctgtt accagtggct   2820
   gctgccagtg gcgataagtc gtgtcttacc gggttggact caagacgata gttaccggat   2880
   aaggcgcagc ggtcgggctg aacggggggt tcgtgcacac agcccagctt ggagcgaacg   2940
   acctacaccg aactgagata cctacagcgt gagctatgag aaagcgccac gcttcccgaa   3000
   gggagaaagg cggacaggta tccggtaagc ggcagggtcg gaacaggaga gcgcacgagg   3060
   gagcttccag ggggaaacgc ctggtatctt tatagtcctg tcgggtttcg ccacctctga   3120
   cttgagcgtc gatttttgtg atgctcgtca ggggggcgga gcctatggaa aaacgccagc   3180
   aacgcggcct ttttacggtt cctggccttt tgctggcctt ttgctcacat gacccgaca    3239

## Description

The invention relates to a process for the regioselective and enantioselective preparation of α-hydroxy carbonyl compounds from α-ketocarbonyl compounds via enzymatic reduction.

### Background of the Invention

Many useful organic compounds, such as pharmaceuticals and food additives, have asymmetric carbon atoms, and enantiomeric forms of these substances exist. In most cases, only one enantiomer is useful as a biologically active substance, whereas the other enantiomer does not show such activity and may even have a harmful effect. Racemic mixtures of such compounds often cannot be used, especially for pharmaceuticals. Therefore, the troublesome resolution of the racemic mixture by means of a conventional organic synthetic process for such optically active substances is unavoidable.

α-oxyfunctionalized carbonyl compounds are indispensable building blocks for asymmetric synthesis due to their versatile functional groups, which are easily transformed to other functionalities, e.g., diols, halo or amino derivatives, and epoxides. α-hydroxy ketones are valuable intermediates and synthons for the production of pharmaceuticals, pheromones and foodstuff. Indeed, optically active α-hydroxy carbonyls have been successfully utilized as chiral auxiliaries and synthons for the asymmetric synthesis of natural products including antitumor agents, antibiotics, pheromones and sugars (Coppola, G.M. and Schuster H. F., Chiral alpha-Hydroxy Acids in Enantioselective Synthesis, Wiley-VCH, Weinheim, Germany (1997); Davis, F.A. and Chen B.C., Chem. Rev. 92:919-934 (1992); Koike, T. et al., Org. Lett. 2: 3833-3836 (2000)).

Ozonolysis, transition metal catalysis, asymmetric enolate oxidation and selective diol oxidation are commonly used chemical methods for the synthesis of α-hydroxy carbonyls. These chemical methods are marred by high production cost, use of toxic and/or harmful reagents that must be removed, high cost, low yield, low regio- and/or stereoselectivity, and extreme temperature of synthesis (Davis, F.A. et al., J. Am. Chem. Soc. 112:6679-6690 (1990); Davis, F.A. and Chen B.C., Chem. Rev. 92:919-934 (1992); D'Accoli, L. et al., J. Org. Chem. 58:3600-3601 (1993); Miller, K.M. et al., J. Am. Chem. Soc. 125:3442-3443 (2003); Gala, D. and DiBenedetto D.J., Tetrahedron Lett. 35:8299-8302 (1994); Saito et al. 1991, Patent EP-A-0482 834; Saito et al. 1993, Patent US005210315A; ; Saito et al. 2007, Patent US20070055081A1).

Various enzymes can be used to produce optically active α-hydroxy carbonyls. Among the first described biotransformations was in Baker's yeast (Csuk, R. and Glänzer, B.I., Chem. Rev. 91:49-97 (1991)). Approaches for the production of α-hydroxycarbonyls using yeast suffer from low yield, low stereo- and/or regioselectivity and narrow substrate spectrum (Kawai, Y., et al., Chem. Soc. Jpn. 72:99-102 (1999); Nakamura et al 1996; Csuk, R. and Glänzer, B.I., Chem. Rev. 91:49-97 (1991); Adam, W. et al., Acc Chem. Res. 32:837-845 (1999)). Bacterial benzoyl formate decarboxylases and benzoyl formate lysases can also produce α-hydroxy ketones, but are limited to the production of aromatic benzaldehyde derivatives and are ineffective when the substrate is *ortho* substituted or when a sterically hindered aryl aldehyde is used (Demir, A.S. et al., Adv. Synth. Catal. 344:96-103 (2002)), significantly limiting their potential. Many other enzymes use kinetic resolution (e.g. transketolases from yeast, lipases and glycerol dehydrogenases) to produce chiral α-hydroxy carbonyls, but the theoretical maximum yield is only 50% with this method (Adam, W. et al., Acc Chem. Res. 32:837-845 (1999)). Enzyme systems such as glycerol dehydrogenases and transketolases, also suffer from enzyme inhibition and/or narrow substrate spectrum (Adam, W. et al., Acc Chem. Res. 32:837-845 (1999)).

Clearly, the convenient and efficient synthesis of optically active α-hydroxy carbonyl compounds is of timely significance and in urgent demand. In essence, the potential of this biocatalytic reaction in synthesizing a library of chiral hydroxy carbonyls is enormous.

In classical organic chemistry the regiospecific reduction of a single keto group from an α-diketone, without reducing the second keto group, is almost impossible, a stereospecific reduction of one of said keto groups is even more problematic. This applies mutatis mutandis to the regiospecific reduction of the keto group in α-ketoaldehydes.

On the other hand, processes for preparing optically active β-hydroxyketone do exist. These protocols are, however, not suitable for the preparation of α-hydroxyketones. Further, enzymes capable of producing α-hydroxy carbonyl compounds are known in the art (Nakagawa, J. et al., J. Biol. Chem. 277:17883-17891 (2002); Bryn, K. et al., Eur. J. Biochem. 18:116-119 (1971)), yet these enzymes are not specific and do not only catalyze the reductions of the desired keto group but also reduce other functional residues (e.g. the second keto group or aldehyde groups).

The acetic acid bacterium *Gluconobacter oxydans* is utilized in large-scale industrial processes for the stereoselective conversion of organic molecules (e.g. in vitamin C production). This conversion is catalyzed by proteins, the so-called oxidoreductases. A number of said oxidoreductases were characterized so far (Deppenmeier, U. et al., Appl. Microbiol. Biotechnol. 60:233-242 (2002)) but none of them react on α-ketocarbonyl compounds.

### Short Description of the Invention

A new class of enzymes was isolated from the acetic acid bacterium *Gluconobacter oxydans* that catalyze regioselective and stereoselective reductions of α-ketocarbonyl compounds (e.g. α-diketones, α-ketoaldehydes and α-keto esters) to form the respective α-hydroxy carbonyl compounds. In case of α-diketones only the carbonyl moiety adjacent to the shorter alkyl chain of the molecule is reduced. α-ketoaldehydes are exclusively reduced at the keto moiety, thus forming only hydroxyaldehydes. A reduction of the chemically more reactive aldehyde moiety does not occur. This applies mutatis mutandis to α-keto esters, which are exclusively reduced at the keto moiety. The enzymes selectively produce the respective optically active isomers, i.e. provide for an enantioselective reduction. Model substrate 2,3-pentanedione was stereospecifically reduced to 2*R*-hydroxy-pentan-3-one by Gox0644, whereas Gox1615 produced 2*S*-hydroxy-pentan-3-one.

All enzymes are dependent on the presence of the cofactor NAD(P)H. A process for regenerating said cofactor was developed, wherein an aldehyde-dehydrogenase converted the NAD(P) back into the reduced form so that the cofactor is only required in catalytic amounts.

These new biocatalysts are innovative tools in the preparation of optically pure key intermediates for pharmaceutical products in current and future industrial processes. The invention thus provides:
(1) a process for the enzymatic regio- and enantioselective production of (R)- and (S)-α-hydroxy carbonyl compounds which comprises reacting an α-ketocarbonyl compound with a *Gluconobacter oxydans* α-ketocarbonyl reductase having the amino acid sequence of Gox0644 (SEQ ID NO:2) or Gox1615 (SEQ ID NO:4) or an addition, substitution, inversion and/or deletion mutant thereof having at least 80% sequence identity with the native reductase of SEQ ID NO: 2 or 4, and reducing the α-keto moiety in α-ketoaldehydes and in α-keto esters and the keto moiety of a vicinal diketone that carries the shorter alkyl chain of the molecule, wherein the reduction of the α-ketocarbonyl compound with Gox0644 (SEQ ID NO:2) or the mutant thereof gives an (R)-α-hydroxy carbonyl compound of the structure: 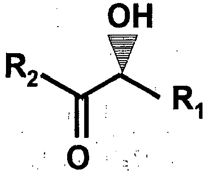 wherein R₁ is alkyl and R₂ is -H, alkyl, aryl, -O-alkyl or -O-aryl, and the reduction of the α-ketocarbonyl compound with Gox1615 (SEQ ID NO:4) or the mutant thereof gives an (S)-α-hydroxy carbonyl compound of the structure: 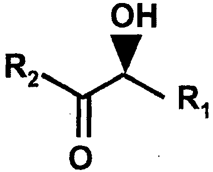 wherein R₁ is alkyl and R₂ is -H, alkyl, aryl, -O-alkyl or -O-aryl;
(2) a preferred aspect of (1) above, wherein a reducing cofactor NAD(P)H is applied in catalytic amounts and is regenerated with acetaldehyde and an aldehyde dehydrogenase;
(3) a preferred aspect of (2) above, wherein the aldehyde dehydrogenase is an aldehyde dehydrogenase derived from *Gluconobacter oxydans;* and
(4) a preferred aspect of (3) above, wherein the aldehyde dehydrogenase derived from *Gluconobacter oxydans* is Gox1122 (SEQ ID NO:6) or a fragment, derivative or mutant thereof.

The biocatalytic process described in the present invention allows an efficient and economic production of the target α-hydroxy carbonyl compounds (S or R isomer) and replaces multi-step processes of classical organic chemistry. Further advantages of the process are its low energy demand, the use of cheap starting products, a reduced demand for hazardous solvents, and easy environmentally friendly access of the reagents (enzymes) from natural sources (microorganisms). Finally, the process provides for the production of α-hydroxy carbonyl compounds, which are quite difficult to be produced by hitherto known classical organic synthesis methods, or are impossible to be produced by such methods.

### Short Description of the Figures

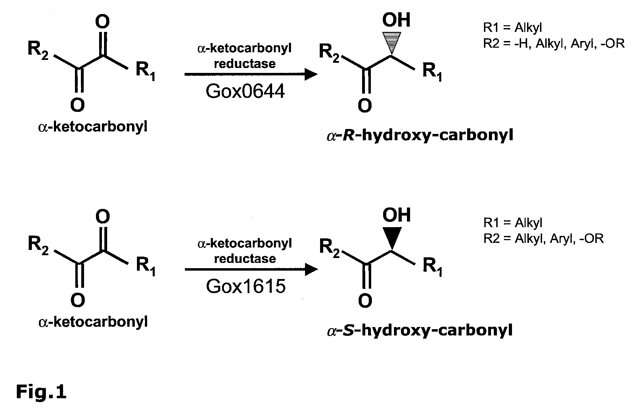

Fig. 1: General scheme of the production of α-(R)-hydroxycarbonyl and α-(S)-hydroxycarbonyl compounds by Gox0644 and Gox1615
Fig. 2: Cloning strategies using BsaI (A) or using BbsI (B).
Fig. 3: NATIVE-PAGE Gox0644 and Gox1615. Lane 1 represents the molecular weight standards of 66 kDa, 140 kDa, 232 kDa, 440 kDa and 669 kDa, lanes 2, 3, Gox1615 and Gox0644, respectively.
Fig. 4: SDS-PAGE analysis of Gox1122, Gox1615, and Gox0644. The proteins were compared to molecular weight marker to determine approximate size (14 kDa, 21 kDa, 30 kDa, 45 kDa, 55 kDa, 66 kDa, 80 kDa, 97 kDa, 116 kDa and 205 kDa molecular weight markers lane 1). Gox1122 (line 2) = 50.5 kDa, Gox1615 (lane 3) = 37.1 kDa, Gox0644 (lane 4) = 31.5 kDa.
Fig. 5: Biotransformation of 2,3-pentanedione (middle spectrum) into 2-hydroxy-pentan-3-one (top spectrum) by Gox0644, tracked by ¹H NMR. The bottom spectrum shows resonances deriving from the coextracted co-substrates/factors of the reaction.
Fig. 6: ¹³C (top) and DEPT135 (bottom) NMR spectra of the biotransformation product 2-hydroxy-pentan-3-one, produced by Gox0644.
Fig. 7: ¹H (top) and ¹³C (bottom) NMR spectra of the biotransformation product 2-hydroxy-pentan-3-one, produced by Gox1615.
Fig. 8: Biotransformation of 1-phenyl-1,2-propanedione (middle spectrum) into 2-hydroxy-1-phenylpropan-1-one (top spectrum) by Gox0644, tracked by ¹H NMR. The bottom spectrum represents a blank sample with resonances deriving from the educt and the coextracted co-substrates/factors of the reaction.

### Sequence Listing - Free Text

### SEQ ID NO: Description

- 1/2: *G. oxydans* α-ketocarbonyl reductase Gox0644 DNA and protein
- 3/4: *G. oxydans* α-ketocarbonyl reductase Gox1615 DNA and protein
- 5/6: *G. oxydans* aldehyde dehydrogenase Gox1122 DNA and protein
- 7-12: primer
- 13: Sequence of pASK-IBA5 expression vector having the following functional features: promoter bp 37-72, forward primer binding site bp 57-76, Strep-tag bp 160-192, multiple cloning site bp 193-274, reverse primer binding site bp 342-358, f1 origin bp 371-809, AmpR resistance gene bp 958-1818 and Tet-repressor 1828-2451.

### Detailed Description of the invention

The genome sequence of *Gluconobacter oxydans* surprisingly revealed various hitherto unknown oxidoreductases. Two such enzymes are preferred aspects of this invention and are particularly suitable for the stereo- and regioselective production of α-hydroxycarbonyl compounds from α-ketocarbonyl compounds.

The process for the enzymatic regio- and enantioselective production of α-hydroxy carbonyl compounds of aspect (1) of the invention comprises reacting an α-ketocarbonyl compound with an α-ketocarbonyl reductase derived from *Gluconobacter oxydans* and being Gox0644 (SEQ ID NO:2), Gox1615 (SEQ ID NO:4), and fragments, derivatives and mutants thereof. These α-ketocarbonyl reductases have an activity to selectively reduce the α-keto moiety in α-ketoaldehydes and/or α-ketoesters or, with respect to α-diketones, the keto group which is proximal to the shorter chain of the molecule. Furthermore, these α-ketocarbonyl reductases show a remarable enantioselectivity, namely they produce the respective R or S enantiomer out of an α-ketocarbonyl compound with an enantiomeric excess (ee) greater than 80, preferably greater than 90 and most preferably greater than 95. These reductases have the following physico- chemical properties:
Gox0644 belongs to the class of aldo-keto reductases and was tentatively referred to as putative 2,5-diketo-D-gluconic acid reductase (Prust, C. et al., Nat. Biotechnol. 23:195-200 (2005)). Here we claim that the enzyme indeed reduced 2,5-diketogluconate but had a broader substrate spectrum with much higher reducing activities towards α-keto carbonyl compounds. Therefore we claim a new application for this enzyme for the regio-and stereospecific production of α-hydroxy-carbonyl compounds. Gox0644 is composed of two identical subunits with molecular masses of 31.6 kDa. The enzyme catalyses the NADPH-dependent reduction of a variety of α-diketones, α-ketoaldehydes and α-keto esters forming α-hydroxyketones, α-hydroxyaldehydes and α-hydroxy esters. Substrates showing the highest activities are 2,3-pentanedione (Km = 5.7 mM, Vmax = 70 U/mg protein), phenylglyoxal (Km = 4.8 mM, Vmax = 75 U/mg protein), phenyl-1,2-propanedione (75 U/mg protein) and ethyl pyruvate (93 U/mg protein). The optimal pH value is 6.8 (7.0) and the optimal temperature for biotransformation is 35 °C. Regio- and stereoselective biotransformation was achieved using 2,3-pentanedione and 1-phenyl-1,2-propanedione. The α-ketocarbonyls were reduced at the keto proximal to the shorter alkyl terminus to produce 2*R-*hydroxy-pentan-3-one and 1-phenyl-2*R*-hydroxy-propanone.

Gox1615 has been cloned, over-expressed in *Escherichia coli*, purified and characterized by Richter et al. (Richter, N. et al., Chem. Bio. Chem. DOI:10.1002/cbic.200900193 (2009)). It is referred to as NADP-dependent glycerol dehydrogenase acting on different aliphatic, branched and aromatic aldehydes. Additionally, the enzyme has been shown to oxidize a variety of different alcohols. Here we claim that the protein has a broader substrate spectrum and reduces α-diketones, α-keto esters, and α-ketoaldehydes with high activities. Hence there is a new application for this enzyme for the regio- and stereospecific synthesis of α-hydroxy-carbonyl compounds. Gox1615 belongs to the class of aldo-keto reductases and is composed of two identical subunits with molecular masses of 37.1 kDa. The enzyme catalyses the NADPH-dependent reduction of a variety of α-diketones, α-keto esters and α-ketoaldehydes forming α-hydroxyketones, α-hydroxy esters and α-hydroxyaldehydes. Substrates showing the highest activities are 2,3-pentanedione and 2,3-hexanedione (Km = 4.0 mM, Vmax = 7.0 U/mg protein), phenylglyoxal and methylglyoxal (Km = 4.3 mM, Vmax = 23 U/mg protein). The optimal pH value is 6.5 and the optimal temperature for biotransformation is 35 °C. α-diketones were regio- and stereoselectivity reduced to 2*S*-hydroxy ketones (e.g. 2*S*-hydroxy-pentan-3-one).

The modifications, i.e. fragments, derivatives and mutants of the aforementioned native enzymes of SEQ ID NOs: 2, 4, 6 include fragments (notably N- and/or C-terminal truncation products), derivatives (notably fusion products with functional proteins and peptides and reaction products with chemical moieties) and mutants (notably addition, substitution, inversion and deletion mutants, having at least 80%, preferably at least 90%, most preferably at least 95% sequence identity with the native enzyme on the amino acid basis or wherein 1 to 20, preferably 1 to 10, consecutive or separated amino acid residues are added, substituted, inverted and/or deleted; for substitution mutants conservative substitution is particularly preferred).

For Gox0644, it is particularly preferred that the modifications are in the following regions (basis numbering shown in SEQ ID NO:2): region 1: 1-10 (N-terminus); region 2: 66-72; region 3: 112-120 (loop between β4 and α4); region 4: 192-202 (loop between β7 and H1); region 5: 266-279 (loop at C-terminus); particularly amino acids K234, R240, W30, A272.

For Gox1615 it is particularly preferred that the modifications are in the following regions (basis numbering of SEQ ID NO:4): region 1: 1-10 (N-terminus); region 2: 321-332 (loop at C-terminus); region 3: 84-90 and region 4: 233-252.

In the process aspect (1) to (3) it is preferred that the reaction is conducted in cell free environment, wherein the α-ketocarbonyl reductase is present as free enzyme or is presented as a cell lysate or as immobilized enzyme. Alternatively the reaction may be conducted in the presence of transformants that produce the α-ketocarbonyl reductase *in situ.* (i.e. a transformant containing a DNA sequence encoding a α-ketocarbonyl reductase as defined hereinbefore)

The α-ketocarbonyl compound that may be reduced is selected from α-diketones, α-keto esters and α-ketoaldehydes. Other α-ketocarbonyl compounds that may be reduced are selected from α-keto acid chlorides and/or α-keto acid anhydrides.

The reaction may be performed in the batch mode or the continuous mode. According to aspect (2) of the disclosure the α-ketocarbonyl reductase is Gox0644 (SEQ ID NO:2) or a fragment, derivative or mutant thereof, and the ketocarbonyl compound to be reduced is an α-diketone, an α-keto ester or an α-ketoaldehyde and the reduction of the α-ketocarbonyl compound gives an (R)-α-hydroxy carbonyl compound of the structure 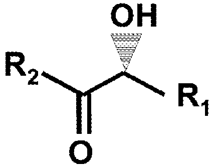 wherein R₁ is alkyl and R₂ is -H, alkyl, aryl, -O-alkyl or -O-aryl.

According to aspect (3) of the disclosure the α-ketocarbonyl reductase is Gox1615 (SEQ ID NO:4) or a fragment, derivative or mutants thereof, and the reduction of the α-ketocarbonyl compound gives an (S)-α-hydroxy carbonyl compound of the structure 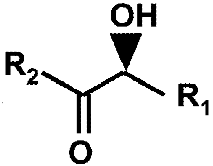 wherein R₁ is alkyl and R₂ is -H, alkyl, aryl, -O-alkyl or -O-aryl.

The alkyl residue in the definition of R₁ and R₂ includes C₁₋₁₀ alkyl residues that might be linear, branched or cyclic and may contain double bonds or substituents (including alkyl, OH, halogen, amino, etc.) and includes methyl, ethyl, n-propyl, iso-propyl, cyclopropyl, n-butyl, sec-butyl, tert-butyl cyclobutyl etc. Particularly preferred are C₁₋₆, more preferred C₁₋₄ alkyl residues.

The aryl residues include mono-, bi- and tricyclic aromatic residues that may be substituted (e.g. with the substituents mentioned for alkyl above). Particular aryl residues include phenyl, toluyl, (1- and 2-)naphthyl, flurenyl, anthracenyl, etc.

Particularly preferred α-hydroxy carbonyl compounds prepared according to the process of aspects (2) and (3) of the invention are 2*R*-hydroxy-pentan-3-one, 1-phenyl-2R-hydroxy-propanone and (*R*)-α-hydroxy carbonyl compounds homologous thereto (such as 2*R*-hydroxy-hexan-3-one, 2*R*-hydroxy-heptan-3-one, 1-phenyl-2*R*-hydroxy-butan-1-one and the like), and 2*S*-hydroxy-pentan-3-one, 1-phenyl-2*S*-hydroxy-propanone and (*S*)-α-hydroxy carbonyl compounds homologous thereto (such as 2S-hydroxy-hexan-3-one, 2S-hydroxy-heptan-3-one, 1-phenyl-2*R*-hydroxy-butan-1-one and the like), respectively. The α-hydroxy carbonyl compounds of the invention (notably the 2R- and 2*S*-hydroxy-pentan-3-one) can be prepared from the respective diketones (2,3-pentadione in case of 2*R*- and 2*S*-hydroxy-pentan-3-one) with ee-values greater than 90, preferably with ee-values greater than 95.

According to aspects (4) and (5) of the disclosure the reducing cofactor NADPH is applied in catalytic amounts and is regenerated with acetaldehyde and an aldehyde dehydrogenase. Such aldehyde dehydrogenase is preferably derived from *Gluconobacter oxydans,* and in particular is Gox1122 (SEQ ID NO:6) or a fragment, derivative or mutant thereof.

Gox1122 is a NADP-dependent aldehyde dehydrogenase and exhibited an apparent molecular mass of 50.1 kDa. The subunit mass is 50.5 kDa, indicating a monomeric structure of the native enzyme. The *Km* value of Gox1122 for acetaldehyde, estimated by using several concentrations of the aldehyde from 0.0025 to 10 mM, is 255 µM. The apparent *Km* value for NADP was 73 µM. The apparent *V*max value, estimated at a fixed 0.5 mM NADP concentration with acetaldehyde as the varied substrate, was 196 U/mg protein. The enzyme also oxidized other short-chained aliphatic and aromatic aldehydes with lower rates. The optimal pH value is 8 and the optimal temperature for biotransformation is 30 °C. No reverse reduction reaction of acetic acid is observed using NADPH a pH range of 4-10. The enzyme is used in a combined enzymatic reaction between the NADPH-dependent reductases (see above) that form α-hydroxy carbonyl compounds. In this combined reaction Gox1122 catalyses the conversion of acetaldehyde into acetic acid and of NADP into NADPH.

As to the mutations of the aldehyde dehydrogenase, refer to the mutations discussed in connection with the reductases above.

The reaction according to aspect (4) and (5) is further explained in the following reaction schemes 1 and 2.

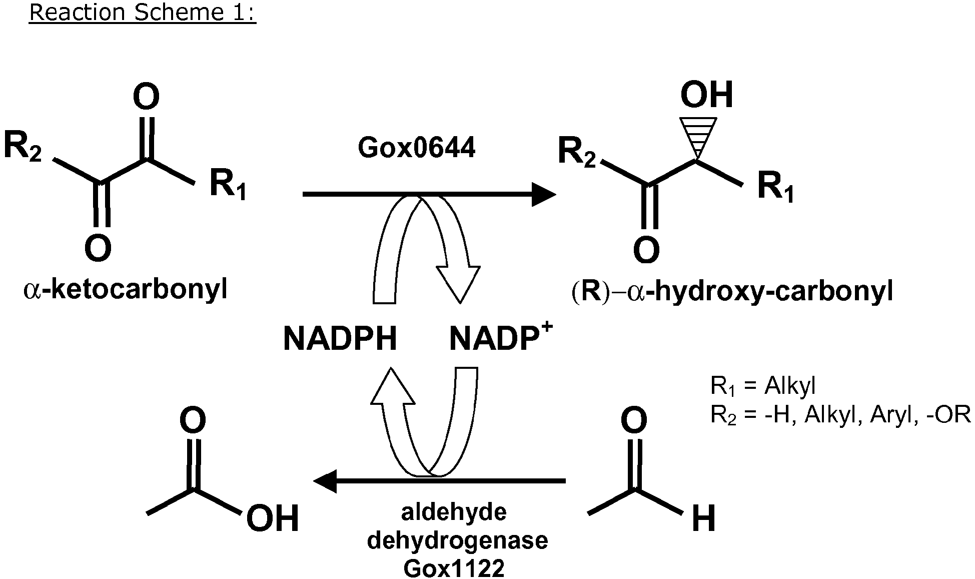

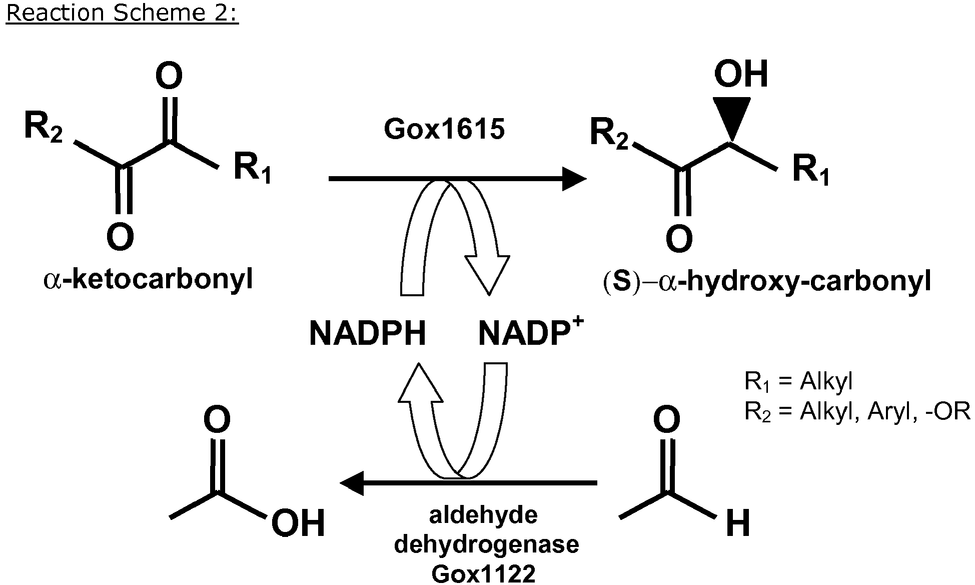

Particular preferred α-ketocarbonyl reductases used in aspects (2) and (3) are those having the sequence of SEQ ID NO: 2 and 4. In this context it is to be noted that Blast searches at NCBI in the databases "Patented protein sequences" and "Non-redundant protein sequences" using the algorithm "blastp" and the matrix "BLOSUM62" were performed for SEQ ID NOs: 2 and 4. There were no homologous proteins (cut off = 77% identity) found for SEQ ID NOs: 2 and 4 in said databases. DNA sequence encoding the abovementioned α-ketocarbonyl reductases or fragments, derivatives or mutants thereof include those having the sequence shown in SEQ ID NOs: and 3. Homologues of said DNA sequences with an identity of greater than 80% on the nucleotide basis are also included. Vectors or expression vectors comprising such DNA sequences are suitable for the production of the α-ketocarbonyl reductases described hereinbefore. Expression vectors strongly depend on the type of the expression host and include that of the pASK-IBA series for the expression in *E. coli* and that of the pEXGOX series (Schleyer, U. et al., Int. J. Food Microbiol. 30:125(1):91-95 (2008)) for (over)expression in *Gluconobacter* species.

Host cells containing at least one of such DNA sequences and/or expression vectors as set forth hereinbefore include any type of prokaryotic host cell; *Gluconobacter* species and *E. coli* are, however, particularly preferred. The α-ketocarbonyl reductase derived from *Gluconobacter oxydans* as defined hereinbefore can be prepared by culturing such a host cell and isolating the α-ketocarbonyl reductase from the culture broth or the host cells. The process may further contain suitable purification steps.

The invention is described in more detail in the following Example, which are however not to be construed as limiting the invention.

### Examples

### Materials and Methods

Microorganisms and culture conditions: *G. oxydans* 621H (DSMZ 2343) was cultivated in a complex medium containing 0.6 % (w/v) yeast extract and 2 % (w/v) mannitol at 30 °C. *E. coli* strains were cultivated in Luria broth (LB) (10 g tryptone, 5 g yeast extract, 10 g NaCl per liter) at 37 °C. For enzyme overproduction *E. coli* DH5α was grown on modified Maximal Induction (MI) medium (Mott, J.E. et al., Proc. Natl. Acad. Sci. USA 82:88-92 (1985)) containing 3.2 % (w/v) tryptone, 2 % (w/v) yeast extract with additions of M9 salts, in addition to 1 mM CaCl₂, 1 mM MgSO₄ and 1 µM FeNH₄ citrate. Ampicillin was added to a final concentration of 100 µg/ml.

Preparation and manipulations of nucleic acids: The plasmid used for cloning and for expression of *gox*0644, *gox*1615, and *gox*1122 in *E. coli* was pASK-IBA5 (IBA GmbH, Goettingen, Germany). The multiple cloning site within the vector contained restriction sites for *BsaI* which allowed the precise fusion of the structural gene with a N-terminal *Strep*-tag^{®} II. The vector also encoded for ampicillin resistance and had a controllable *tet A* promoter/repressor system. The pASK-IBA5 expression vector (SEQ ID NO:13) contains a strong regulatable Tet promoter, start and stop codon, ribosomal binding site, the Strep-tag II sequence, and restriction endonuclease sites within the multiple cloning sequence (MCS). Furthermore, the vector has been optimized for protein expression in *E. coli* strains, generating large amounts of homologous recombinant protein.

Plasmid DNA was prepared from *E. coli* with GeneJET Plasmid Miniprep Kits (Fermentas GmbH). A 3 ml starter culture of *E. coli* was grown overnight in LB-medium containing the respective antibiotic at 37 °C, was transferred to sterile microfuge tubes and centrifuged at 12,000 rpm for 5 min (Biofuge 15, Heraeus GmbH, Osterode, Germany). The cell pellet was taken up in 250 µl resuspension solution (Fermentas GmbH), 250 µl lysis solution (Fermentas GmbH) was added and shaken until a clear solution was obtained. The solution was neutralized by mixing with of 350 µl neutralization solution (Fermentas GmbH). After centrifugation at 12,000 rpm for 5 min, the supernatant was transferred to the GeneJET mini-column, which was subsequently centrifuged at 12,000 rpm for 30s. The primary eluate was discarded. The column was then treated twice with 500 µl wash solution (Fermentas GmbH), wherein the solution was applied to the column and centrifuged at 12,000 rpm for 30 s. The eluate was again discarded. The plasmid DNA was eluted from the column by applying 50 µl elution buffer (Fermentas GmBH) at pH 8,0. After incubation for 2 min the column was centrifuged (12000 rpm, 1 min) and the eluted DNA was stored at 4 °C.

DNA Isolation: Total DNA from *G. oxydans* was isolated by the CTAB method (Ausubel, F.M. in Ausubel, Brent, Kingston, Moore, Seidman and Struhl (eds): Current Protocols in Molecular Biology, John Wiley & Sons, Inc., New York, Vol 5: pp 2-11 (2002)). *G. oxydans* was cultured in 50 ml complex medium up to the stationary phase. The cells were sedimented by centrifugation (8,000 rpm, 4 °C, 15 min SS 34-Rotor in a Sorvall centrifuge RC-5B (Fa. Du Pont, Bad Homburg) and the pellet was resuspended in 0.95 ml TE-buffer (Tris/HCl 10 mM and EDTA 1 mM, pH 8.0). After addition of 50 µl 10 % (w/v) SDS und 5 µl proteinase K solution (20 mg/ml) the mixture was incubated at 37 °C for 1 h. Thereafter 0.18 ml 5 M NaCl and 0.15 ml CTAB/NaCl solution (700 mM NaCl and 275 mM CTAB) were added, mixed and the suspension was kept in a water bath at 65 °C for 2 h. For extraction of the proteins 0.133 ml (1 vol.) phenol/CHCl₃ 24:1 (v/v) was added and carefully mixed. After centrifugation the upper aqueous phase was extracted with 0.5 vol. CHCl₃/isoamylalcohol 1:1 (v/v). For precipitation of DNA 0.6 vol. isopropanol was added to the resulting aqueous phase followed by centrifuging at 6,000 rpm, 4 °C for 15 min. The pellet was washed with 0.1 ml cold 70 % (v/v) aqueous ethanol, dried for 10 min at 60 °C and subsequently taken up in 0.2 ml TE-buffer with vortexing. Thereafter the DNA was incubated for 10 min on a water bath at 70° C, centrifuged and was pipetted to obtain a homogenous viscous solution. The DNA was stored at 4 °C.

Polymerase chain reaction (PCR), Primer Construction: Primers for PCR were constructed using the program Primer D'Signer 1.1 (IBA GmbH, Goettingen, Germany). The oxidoreductase genes were amplified by PCR with the introduction of BsaI endonuclease sites to complement the digested plasmid vector (see below). Alternatively, *BsmBI* or *BbsI* could be introduced at the ends of the PCR fragments when the genes contained internal *BsaI* sites. Therefore, the primers for all the soluble dehydrogenase/oxidoreductase genes were constructed with either, *BsaI, BsmBI* or *BbsI* restriction endonuclease sites introduced at both ends of the gene (see Table 1 below).

The primers with *Bsa*I restriction sites were for *gox*0644 and *gox*1122. The primers for *gox*1615 contained *BsmBI* restriction sites.

**Table 1: Primers used for PCR**

| Primer (SEQ ID NO) | Primer sequence | Restriction site introduced |
|---|---|---|
| GOX0644_5f (7) | ATGGTAGGTCTCAGCGCCTCGTCACAGGTTCCATCCGCC | *BsaI* |
| GOX0644_5r (8) | ATGGTAGGTCTCATATCAGAATTTCGCCGTATTCGGATCAG | *BsaI* |
| GOX1615_5f (9) | ATGGTACGTCTCAGCGCCGCATCCGACACCATCCGCATC | *BsmBI* |
| GOX1615_5r (10) | ATGGTACGTCTCATATCAGTCCCGTGCCGGGGGCGC | *BsmBI* |
| GOX1122_5f (11) | ATGGTAGGTCTCAGCGCCGCTTACGCTACGATCAACCCTTA | *BsaI* |
| GOX1122_5r (12) | ATGGTAGGTCTCATATCAGAACGGCGCGTCGATATCAACA | *BsaI* |

PCR conditions: The amplification reaction mixtures contained 100 ng of total DNA of *G. oxydans,* 0.5 pmol of each oligonucleotide primer, 200 µM each deoxynucleotide triphosphate (dNTP), 5 µl of 10X *Pfu* cloned polymerase buffer, and 2.5 U of *Pfu* (Stratagene, La Jolla, CA, USA) in a total volume of 50 µl. The mixture was subjected to PCR amplification in a DNA thermal cycler (Bio-Rad MyCycler) for 30 cycles. Each cycle was defined by a 45-second denaturing step at 95 °C, a 1-minute annealing step at 55 °C, and a 2.5-minute polymerization step at 72 °C. PCR products were analyzed by electrophoretic separation in a 0.8 % agarose gel in a Tris-acetate buffer system. PCR fragments were microdialyzed with 0.025 µm membrane filters for 1 h.

Clone Development: After generation of PCR products the DNA fragments were cloned into the pASK vector. *Bsa*I and *BsmBI* belong to the Type IIS restriction enzymes that cleave the DNA outside their recognition sites. Thereby, the digestion with a single enzyme can generate two different independent sticky ends with 4-base 5'-overhangs allowing unidirectional cloning. Each of these three restriction endonuclease recognize a different cut sequence, but all produce the same 5' to 3' overhang consisting of GCGC that precisely complements the 3' to 5' pASK-IBA5 overhang of CGCG. At the 3' end of the gene the restriction enzymes produce the sequence TATC, which is complementary to the 3' to 5' sequence ATAG on the plasmid.

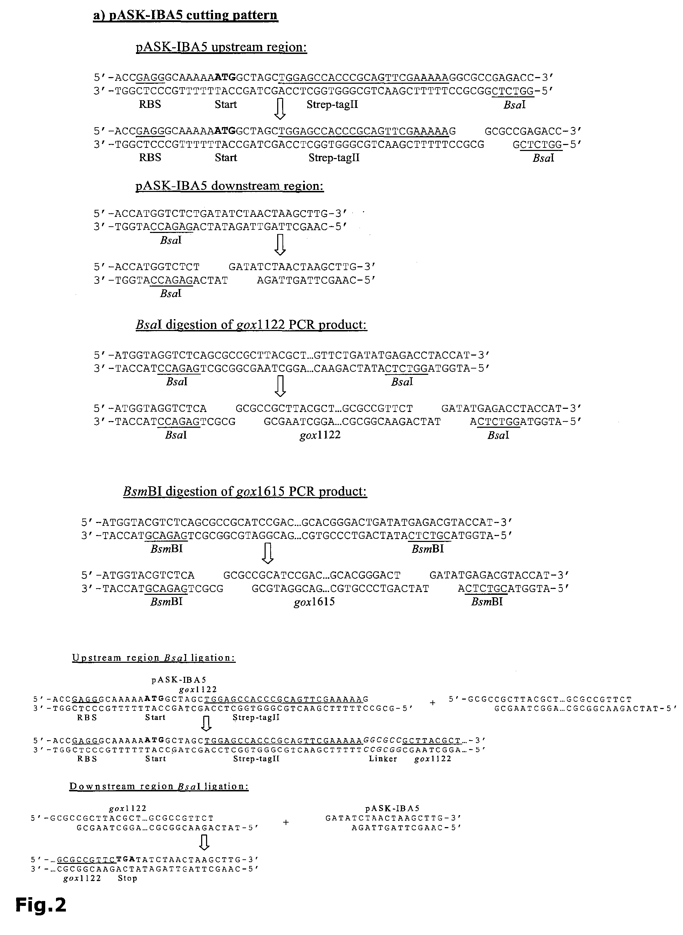

Vector pASK-IBA5 was digested with *BsaI* for cloning of restriction endonuclease treated PCR products representing genes *gox*0644, and *gox*1122. The cloning strategy is shown in Fig. 2. Fig. 2A describes the sequence complementation of *gox*0644 after restriction digestion with *BsaI. gox*0644 represents all genes using *BsaI* cut sites producing the 5' CGCG 3' sequence and 5' TATCA 3' on the reverse strand, which allows for precise ligation into the pASK vector. Fig. 2B describes the sequence complementation of gox1615 after restriction digestion with BsmB1 producing the 5' CGCG 3' sequence and 5'TATCA 3' on the reverse strand, which allows for precise ligation into the pASK vector.

Successful incorporation of the PCR oxidoreductase gene construct into the pASK IBA vector was achieved according to the ligation protocol using T4 ligase (New England Biolabs, Ipswich, MA, USA). For this procedure 2 µl T4 ligase buffer was mixed with 2 µl BsaI digested pASK5, 15 µl BsaI (or BbsI or BsmBI as indicated in Tab. 1) digested PCR product and 1 µl of T4 ligase. The mixture was incubated for 1 hour at room temperature. This procedure incorporates the restriction digested gene of interest with a 5' overhang sequence GCGC to the pASK-IBA5 vector with the complementary sequence overhang. The 3' overhang of the gene containing the GATA sequence was fused to the complementary overhanging sequence (Fig. 2A-B Schemes of cloning strategies). This protocol achieved a high success rate of ligation based on the high ratio of restriction endonuclease (*BsaI, BsmBI*) cut purified PCR product to *BsaI* cut plasmid.

Once ligated the plasmids containing the inserts were transformed into competent *E. coli* DH5α cells (Inoue, H. et al., Gene. 96:23-28 (1990)). Competent *E. coli* cells containing the plasmid with the recombinant oxidoreductase genes were allowed to outgrow in 500 µl of LB media for one hour at 37 °C. Incremental aliquots of 20 µl, 50 µl and 150 µl of *E. coli* DH5α cells were plated on LB plates containing 1 µg/ml ampicillin and incubated at 37 °C to insure growth with adequate separation between colonies for easy colony selection.

Colonies were randomly chosen and grown overnight at 37 °C in LB broth containing 1µg/ml of ampicillin to validate the correct incorporation of the gene of interest in the pASK plasmid. Plasmids were purified as described above. To check all aspects associated with the plasmid construct the following plasmid components: Strep TagII, ribosome binding site, linker sequence, start codon, termination codon and a gene insert ligated in the proper reading frame with the start codon, were analyzed by sequencing the corresponding region of the plasmid at the Madison Biotechnology Sequencing center.

Overexpression: Overnight starter cultures of the validated stock pASK-IBA5 oxidoreductases in *E. coli* DH5α were grown at 37°C in 20ml LB medium containing 20 µg of Ampicillin. Culture flasks with 80% headspace provided optimal aeration at shaker speeds of 200 rpm or greater, for the overexpression of heterlogous proteins. These starter cultures of actively replicating cells were used to generate rapid cell growth at 37 °C when added to yeast extract and tryptone containing Maximal Induction media (MI media in methods). Ampicillin (1 µg/µl) was again added to inhibit ampicillin sensitive organisms. *E. coli* growth and protein expression was optimized on MI media through a biphasic production system. The first phase of growth supported cellular replication, in which heterologous protein production was inhibited at the tetracycline promoter (tet promoter) by the tetracycline repressor protein. The second phase focused on the optimized expression of heterologous protein in which available resources were predominantly directed toward protein synthesis. Protein synthesis was initiated by the addition 100 µl of 2 mg/ml anhydrotetracycline stock solution for 1 I of cell culture when the culture density reached an OD₆₀₀ of 0.4-0.6. The cells were allowed to grow for an additional four hours to complete protein production Cells were harvested by centrifugation at 4500 x g at 4 °C. Cell pellets were resuspended in 15 ml Buffer W (100 mM Tris/HCl pH 8.0, 150 mM NaCl) at 4 °C. Protease cocktail set III (10 µl/l cells) (Merck KGaA, Darmstadt, Germany) and DNase I (20 U/I cells) (Abgene, Epsom, UK) were added to the resuspended cell pellets and allowed to incubate on ice for ten minutes. Resuspended cells were lysed by sonication at 4 °C (30-35W for 10 s intervals over 10 min). Lysates were centrifuged at 24,000 x g at 4 °C and the cleared lysate was collected.

At this point the overexpressed soluble oxidoreductase was suspended in the buffer along with other soluble cellular proteins necessitating further purification via the Strep Tag II purification system. Column chromatographies were carried out at 4 °C with a Strep-Tactin Superflow^{®} affinity column (IBA GmbH, Goettingen, Germany) connected to an ÄKTA-FPLC system (Amersham Bioscienes, Piscataway, NJ, USA). A bed volume of 10 ml was equilibrated with three column volumes of Buffer W. Afterwards the cleared cell lysate containing the Strep Tag II fused soluble oxidoreductases was applied and the column was washed with five column volumes of Buffer W. To elute the bound protein buffer E (buffer W containing 2.5 mM desthiobiotin) was applied to the column. After collection of the pure biocatalysts the affinity column was regenerated with buffer R (buffer W containing 1 mM HABA).

### Characterization of the Isolated Enzymes:

*Polyacrylamide gel electrophoresis (PAGE):* SDS-PAGE was done on a 14 % (w/v) slab gel as described by Laemmli (Laemmli, U.K., Nature 227: 680-685 (1970)) with 4 % (w/v) polyacrylamide stacking gel. Before application samples were diluted 1:1 (v/v) in sample loading buffer (2 % [w/v] SDS, 5 % [v/v] β-mecaptoethanol, 50 % [v/v] glycerol, 20 % [v/v] collecting buffer pH 6.8, 0.001 % [w/v] bromophenol blue) and boiled for 10 min. The following reference proteins (Amersham Bioscienes, Piscataway, NJ, USA) with the indicated molecular masses were used for the measurement of the molecular mass of cytosolic oxidoreductases: myosin (205 kDa), β-galactosidase (116 kDa), phosphorylase b (97 kDa), transferrin (80 kDa), bovine serum albumin (66 kDa), glutamate dehydrogenase (55 kDa), ovalbumin (45 kDa), carbonic anhydrase (30 kDa), and lysozyme (14 kDa).

*Molecular Sieve Chromatography:* A Sephacryl 300HR 16/60 chromatography (Amersham) with reference proteins Blue Dextran (2 MDa), Thyroglobulin (669 kDa), Ferritin (440 kDa), Catalase (232 kDa), Aldolase (140 kDa) and Ovalbumin (43 kDa) were used to determine the native weight of the aldehyde dehydrogenases. The native molecular mass for each protein was determined by comparison to the standard curve generated by the molecular weight standards that were applied to a gel filtration column. The distances each molecular weight standard traveled (Rf) was plotted against the logarithm of the molecular mass of the standards.

*Determination of pH and temperature optima and enzyme kinetics:* The optimum pH for enzyme activities was determined by a modified method of Salusjärvi, T. et al., Appl. Microbiol. Biotechnol. 65:306-314 (2004) using the following buffer system (50 mM final concentration each): sodium acetate pH 4.5 - 6.5, potassium phosphate pH 6.5 - 8.0, Tris-HCl pH 8.0 - 10. Temperature optima for the biocatalysts were determined under standard reaction conditions at temperatures ranging from 20 - 80 °C. The Michaelis constant (Km) for the proteins was determined from purified enzyme by Lineweaver-Burk plots. Assays were performed at optimum pH for each enzyme and at 30 °C using the best substrate and a targeted substrate. The Km for each substrate was calculated using concentrations of 0.0025 to 10 mM, and the Km NAD(P)H and NAD(P) was calculated using concentrations of 0.01 to 0.5 mM

*Measurement of enzyme activity:* NADPH-dependent α-ketocarbonyl reductases were assayed by a routine method used for NAD(P)-linked enzymes by recording the rate of decrease of NADPH at 340 nm (ε = 6.22 mM⁻¹ x cm⁻¹)) with substrates at 30 °C. The reaction mixture (1 ml) for reduction of α-ketocarbonyl substrates contained 10 mM substrate, 40 mM potassium phosphate buffer pH 7.0, 0.025 mM NAD(P)H and the enzyme. Reactions were induced by addition of enzyme or substrate. One unit was defined as the amount of enzyme activity catalyzing the conversion of 1 µmol of pyridine nucleotide per min at 30 °C. *NMR Analysis of Biotransformation Products:* Biotransformations were extracted with equal volumes of CDCl₃. and ¹³C NMR spectra were recorded on a Bruker Avance 300 DPX spectrometer operating at 300 MHz for ¹H NMR and at 75 MHz for ¹³C NMR, respectively. Spectra were processed using Bruker TonSpin 1.3 for Windows software and calibrated to the residual solvent peak of CDCl₃ (δ_{H} = 7.26 / δ_{C} = 77.0).

*2,3-pentanedione:* ¹H NMR (CDCl₃) δ: 1.05 t, *J*=7.3 Hz (H₃-5); 2.31 s (H₃-1); 2.75 q, *J*=7.3 Hz (H₂-4).

*2-hydroxy-pentan-3-one:* ¹H NMR (CDCl₃) δ: 1.11 t, *J*=7.4 Hz (H₃-5); 1.37 d, *J*=7.0 Hz (H₃-1); 2.51 m (H₂-4); 4.24 q, *J*=7.0 Hz (H-2); 3.61 d, *J*=4.4 Hz (OH). ¹³C NMR (CDCl₃) δ: 7.5 (CH₃-5); 19.9 (CH₃-1); 30.7 (CH₂-4); 72.4 (CH-2); 213.1 (C-3).

*1-phenyl-1,2-propanedione:* ¹H NMR (CDCl₃) δ: 2.52 s (H₃-3); 7.49 dddd, *J*=8.1, 7.4, 0.7, 0.0 Hz (H-3' + H-5'); 7.64 tt, *J*=7.4, 1.5 Hz (H-4'); 8.00 dddd, *J*=7.0, 1.5, 1.0, 0.0 Hz (H-2' + H-6').

*1-phenyl-2-hydroxy-propanone:* ¹H NMR (CDCl₃) δ: 1.45 d, *J*=7.0 Hz (H₃-3); 3.82 d, *J*=6.6 (OH); 5.16 quint *J*=6.6; 7.50 dddd, *J*=8.5, 7.9, 1.2, 0.0 Hz (H-3' + H-5'); 7.62 tt, *J*=7.4, 1.5 Hz (H-4'); 7.92 dddd, *J*=8.3, 1.4, 0.9, 0.0 Hz (H-2' + H-6').

*Determination of the absolute configuration of the biotransformation product 2-hydroxy-pentan-3-one using chiral NMR shift solvents:* Chiral NMR shift reagent (*R,R*)- and (*S,S*)-bis-α-methylbenzylamine-para-methyl (BMBA-*p*-Me) was prepared with small modifications according to the method reported by Kobayashi, Y et al., Tetrahedron Lett. 44: 7489-7491 (2003). In brief, the method of Marshall, J.A. and Mikowski, A. M., Org. Lett. 8:4375-4378 (2006) was used to synthesize 1,3-dibromo-2-methylpropane from 1,3-dihydroxy-2-methylpropane. Diisopropylethylamine (25.5 g), triphenylphosphine (20.1 g) and CBr₄ (25.5 g) were sequentially added to a stirring solution of 1,3-dibromo-2-methylpropane (2.2 g) in 100 ml CH₂Cl₂ at 0 °C. The reaction was stirred for 3-4 h at 0 °C and concentrated under reduced pressure to give a green-brown oil. The oil was dissolved in diethyl ether and water (100 ml each) and the aqueous layer was extracted three times with diethyl ether. The organic extracts were combined and washed with water and brine, and dried over MgSO₄. The reaction was concentrated under reduced pressure and applied to flash chromatography on silica 60 (230-400 mesh, 100% cyclohexane). Fractions containing the 1,3-dibromo-2-methylpropane product were combined (as judged by Si-TLC [100% cyclohexene]) and concentrated under reduced pressure resulting in a brown-yellow oil. The dibromopropane oil was used for the synthesis of (*R,R*)- and (*S,S*)-BMBA-*p*-Me as described by (Kobayashi, Y. et al., Org. Lett. 4(3):411-414 (2002). The crude oil was distilled by Kugelrohr at 240 °C to give crude BMPA-*p-*Me (yellow oil). BMBA-p-Me was purified by HPLC using a C18 column (Waters Xterra, 250 x 4.6 mm) at a flow rate of 0.8 ml min⁻¹ and a 40 min linear gradient of 50-100 % MeOH at ambient temperature. Fractions were concentrated under reduced pressure and BMBA-*p*-Me was identified by ¹H-NMR (colorless hygroscopic oil).

Approximately 7.5 mg of the extracted product 2-hydroxy-pentan-3-one was added to 15 mg (*R,R*)- or (*S,S*)-BMBA-*p*-Me (dissolved in 200-300 µl CDCl₃) and analyzed by ¹³C NMR with the readout adjusted to 0.001 ppm/point (sw = 239 ppm, td = 512k) (Fiorentino, A. et al., Tetrahedron., 62:8952-8958 (2006)). The absolute configuration was determined based on the sign of Δδ*_{RR-SS}* (δ_{(*R,R*)-BMBA-*p*-Me} - δ_{(*S,S*)-BMBA-*p*-Me}) for the α-carbons adjacent to the hydroxymethine unit as formulated in the following model shown in Scheme 3 (valid for saturated secondary alcohols of acyclic and cyclic compounds):

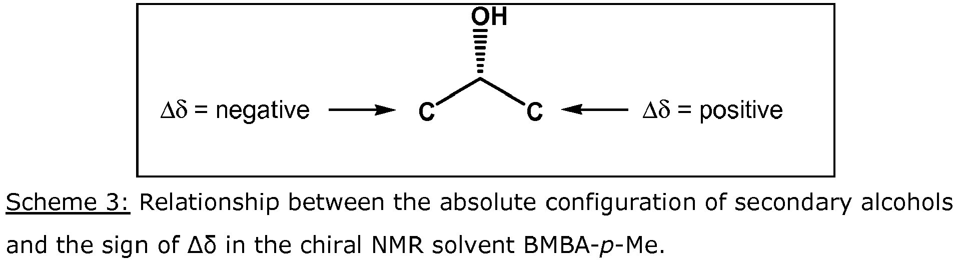

*Cofactor regeneration:* A prerequisite for the production of α-hydroxycarbonyl compounds is the regeneration of the expensive coenzymes and the stability of the protein catalysts. By coupling two coenzyme-dependent enzyme reactions, NADPH can be continuously regenerated. Hence, instead of stoichiometric amounts, only catalytic amounts of coenzyme are required. For this purpose, we used a combined enzymatic reaction between the NADPH-dependent oxidoreductases that form α-hydroxycarbonyl compounds and the aldehyde dehydrogenase GOX1122 (see Schweiger, P. et al., J. Mol. Microbiol. Biotechnol. 13:147-155 (2007)) that produces the reduced cofactor in the course of acetaldehyde oxidation. The aldehyde dehydrogenase has very high turn-over rates and low Kₘ values for both acetaldehyde and NADP and could be produced in high amounts (Schweiger, P. et al., J. Mol. Microbiol. Biotechnol. 13:147-155 (2007)). The biotransformation was performed in a batch process (total volume 2.5 ml 40 mM potassium phosphate buffer pH 7). 30 µg of purified Gox1122 and of NADPH-dependent oxidoreductases and 50 µM NADPH were added. The substrate concentration in the beginning was 20 mM 2,3-pentanedione and 20 mM acetaldehyde. Fresh substrates were added every 60 min (20 mM each).

### Example 1: Isolation and preparation of the biocatalysts

The isolation and production of the biocatalysts, Gox0644 and Gox1615, which are capable of selectively reducing α-ketocarbonyl compounds into α-hydroxycarbonyl compounds is described in detail in the following. The isolation of Gox1122 for the cofactor regeneration is disclosed in Schweiger, P. et al., J. Mol. Microbiol. Biotechnol. 13:147-155 (2007).

1.1. PCR amplification of genes *gox*0644 *and gox*1615: Oligonucleotides containing extended 5' *Bsa*I, or *BsmBI* restriction sites that correspond to *gox*0644, *and gox*1615 of *G. oxydans* were constructed by utilizing the primers of Table 1. The amplification reaction mixtures contained 100 ng of total DNA of *G. oxydans,* 0.5 pmol of each oligonucleotide primer, 200 µM each deoxynucleotide triphosphate (dNTP), 5 µl of 10X *Pfu* cloned polymerase buffer, and 2.5 U of *Pfu* (Stratagene, La Jolla, CA, USA) in a total volume of 50 µl. The mixture was subjected to PCR amplification in a DNA thermal cycler (BioRad MyCycler) for 30 cycles. Each cycle was defined by a 45-second denaturing step at 95 °C, a 1-minute annealing step at 58 °C, and a 2.5-minute polymerization step at 72 °C. PCR products were analyzed by electrophoretic separation in a 0.8 % agarose gel. PCR fragments were microdialyzed with 0.025 µm membrane filters for 1 h.

1.2. Cloning of genes: Vector pASK5-IBA5 contains a N-terminal eight amino acid Strep-tag II that is precisely fused to the structural gene. PCR products and plasmid pASK-IBA5 were subjected to *BsaI* or other appropriate restriction digestion (New England Biolabs, Ipswich, MA, USA). Ligation of the digested vector and PCR fragment was performed with T4 ligase (New England Biolabs, Ipswich, MA, USA), transformed into *E. coli* DH5α cells and plated on LBₐₘₚ selection plates. Plasmids displaying proper digestion patterns were sequenced to confirm correct gene insertion of *gox*0644 and *gox*1615, respectively.

1.3. Overexpression and Purification of oxidoreductases: Whole seed cultures of *E. coli* BL21 or *E. coli* DH5α containing plasmid constructs of interest were used to inoculate 1 l MI medium. Cultures were grown at 37 °C and induced by addition of 200 ng/ml anhydrotetracycline at an OD₆₀₀ = 0.4, and allowed to grow for an additional four hours. Cells were harvested by centrifugation at 4500 x g at 4 °C. Cell pellets were resuspended in 15 ml Buffer W (100 mM Tris/HCl pH 8.0, 150 mM NaCl) at 4 °C. Protease cocktail set III (10 µl/l cells) (Merck KGaA, Darmstadt, Germany) and DNase I (20 U/I cells) (Abgene, Epsom, UK) were added to the resuspended cell pellets and allowed to incubate on ice for ten minutes. Resuspended cells were lysed by sonication at 4 °C. Lysates were centrifuged at 24,000 x g at 4 °C and the cleared lysate was collected. Column chromatographies were carried out at 4 °C with a Strep-Tactin Superflow^{®} affinity column (IBA GmbH, Goettingen, Germany). A bed volume of 10 ml was equilibrated with three column volumes of buffer W. Cleared lysates were centrifuged at 12,000 x g at 4 °C for 10 minutes prior to application to the column. The column was washed with five column volumes of Buffer W after the cell extract entered the column. To elute the bound protein Buffer E (Buffer W containing 2.5 mM desthiobiotin) was applied to the column. Elution of the enzymes occurred between 20 -30 ml of Buffer E application.

NADPH-dependent α-ketocarbonyl reductases were assayed by a routine method used for NAD(P)-linked enzymes by recording the rate of decrease of NADPH at 340 nm (ε = 6.22 mM^{-1.}cm⁻¹) with substrates at 30 °C. The reaction mixture (1 ml) for reduction of α-ketocarbonyl substrates contained 10 mM substrate, 40 mM potassium phosphate buffer pH 7.0, 0.025 mM NADPH, and the enzyme. Reactions were induced by addition of enzyme or substrate. One unit of activity was defined as the amount of enzyme activity catalyzing the conversion of 1 µmol of pyridine nucleotide per min at 30 °C.

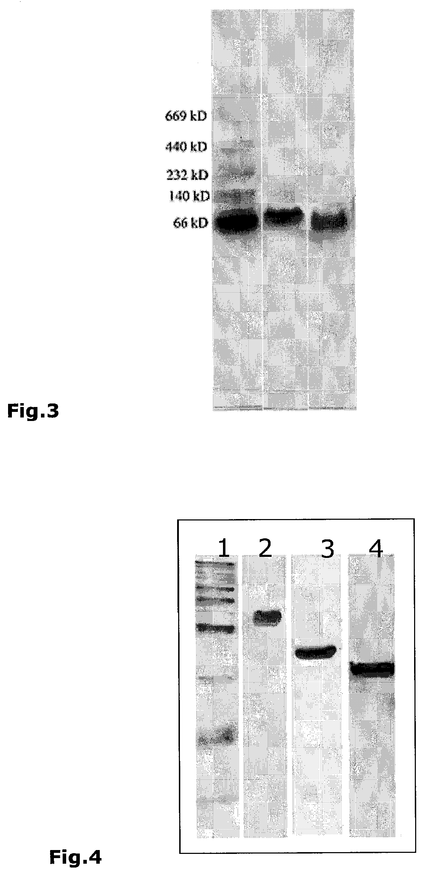

Native enzymes Gox0644 and Gox1615 showed single bands when applied to a native PAGE. The peaks corresponded to 66.1 kDa for Gox0644 and 74.5 kDa for Gox1615 (Fig. 3). These data suggest that both Gox0644 and Gox1615 from *G. oxydans* 621H are active as homodimers.

The proteins were also analysed by SDS-PAGE to verify the expected molecular masses of the single subunits by comparison to molecular weight markers. The results are shown in Fig.4. 14 kDa, 21 kDa, 30 kDa, 45 kDa, 55 kDa, 66 kDa, 80 kDa, 97 kDa, 116 kDa and 205 kDa molecular weight markers lane 1; Gox1122 (lane 2) = 50.5 kDa; Gox1615 (lane 3) = 37.2 kDa and Gox0644 (lane 4) = 31.7 kDa. These results were then compared to the expected sizes derived from the known amino acid sequence. The SDS-PAGE results verified the purity of the protein sample through the visualization of a single pure band on the gel. The biocatalysts all correlated to the expected molecular mass (Tab. 2).

**Table 2: Final Molecular Mass and Subunit Composition**

| Protein | Subunit Mass | Determined Native Mass¹ | Subunit Composition |
|---|---|---|---|
| Gox0644 | 31.6 | 66.1 | Dimer |
| Gox1615 | 37.1 | 74.5 | Dimer |
| Gox1122 | 50.5 | 50.2 | Monomer |

| | | | |
|---|---|---|---|
| 1) final subunit composition of each protein in their native state. | | | |

### Example 2: Characterization of the biocatalysts

For each enzyme (Gox0644 and Gox1615, respectively), fresh enzyme preparations were used to validate the substrate spectrum and the kinetic activity. Each protein exhibited activity with α-ketoaldehydes, α-keto esters and/or α-diketones (Tab. 3) that are a special class of organic compounds capable of chiral product formation through regioselective and stereospecific reduction.

Purified NAD(P)-dependent oxidoreductases were assayed at 30 °C by a routine method used for NAD(P)-linked enzymes by recording the rate of decrease of NAD(P)H at 340 nm (ε = 6.22 mM⁻¹˙cm⁻¹) with substrates capable of reduction reactions. The standard reaction mixture (1 ml) for the reduction reactions of substrates contained 10 mM substrate, 40 mM potassium phosphate buffer (K-phosphate buffer) pH 7.0, 0.05 mM NAD(P)H, and the enzyme unless otherwise noted. Reactions were induced by addition of enzyme or substrate. One unit of activity was defined as the amount of enzyme activity catalyzing the conversion of 1 µmol of pyridine nucleotide per min at 30 °C. Measurement of the protein content was performed by the method of Bradford (Bradford M.M., Anal. Biochem. 72:248-254 (1976)).

**Table 3: Enzymatic activities with α-diketones, α-ketoaldehydes and α-ketoesters**

| Substrate | Gox0644 U/mg* | Gox1615 U/mg* |
|---|---|---|
| α-diketones | | |
| 2,3-pentanedione | 69.6 | 7.0 |
| 2,3-hexanedione | 50.1 | 6.5 |
| 3,4-hexanedione | 7.3 | 2.0 |
| 1-phenyl-1,2-propanedione | 75.4 | 5.4 |
| α-ketoaldehydes | | |
| phenylglyoxal | 74.7 | 19.1 |
| methylglyoxal | 3.4 | 23.0 |
| α-ketoester | | |
| isoamyl pyruvate | 63.1 | 4.7 |
| ethyl pyruvate | 92.8 | 1.2 |

| | | |
|---|---|---|
| * U/mg = U/mg protein = µmol x min⁻¹ x mg protein⁻¹ | | |

2.1. Enzymatic activity of Gox0644: Gox0644 utilized NADPH in the reduction reactions of α-diketones, α-ketoaldehydes and α-keto esters. The α-diketones, 2,3-butanedione, 2,3-pentanedione, 2,3-hexanedione and 1-phenyl-1,2-propanedione displayed the highest activity (Tab. 4). The α-ketoaldehydes phenylglyoxal and methylglyoxal and the keto ester ethyl pyruvate also exhibited high activity. Particular substrates not reduced by Gox0644 were acetoin, 2-oxo butyric acid, acetylacetone, acetone, and simple aldehydes. The results show that Gox0644 carbonyl reduction is unable to achieve activity with β-diketones, β-veto acids from fatty acid degradation or with 2-keto acids associated with amino acid catabolism. Furthermore, the results with acetoin reduction or oxidation did not display activity. These results show that the α-diketones reaction selectively reduced only one carbonyl group and was irreversible.

**Tab. 4: Enzymatic activity of Gox0644**

| Substrates | Specific Activity (U/mg)* | Cofactor |
|---|---|---|
| glyoxal | 1.1 | NADPH |
| methylglyoxal | 3.4 | NADPH |
| 2,3-butanedione | 60.0 | NADPH |
| 2,3-pentanedione | 69.6 | NADPH |
| 2,3-hexanedione | 50.1 | NADPH |
| phenylglyoxal | 74.7 | NADPH |
| 3,4-hexanedione | 7.3 | NADPH |
| ethyl pyruvate | 92.8 | NADPH |
| 1-phenyl-1,2-propanedione | 75.4 | NADPH |
| benzil | 14.0 | NADPH |
| isoamyl pyruvate | 63.1 | NADPH |

| | | |
|---|---|---|
| *U/mg = µmol/mg˙min | | |

2.2. Enzymatic activity of Gox1615: Gox1615 revealed activity with wide variety of chemical substrates. As can be seen from Tab. 5, Gox1615 specifically used NADPH and displayed the highest activity with the ketoaldehydes phenylglyoxal and methylglyoxal, but also utilized diketones and aldehydes.

**Tab. 5: Enzymatic activity of Biocatalyst Gox1615**

| Substrates | Specific Activity (U/mg)* | Cofactor |
|---|---|---|
| glyoxal | 5 | NADPH |
| methylglyoxal | 23.0 | NADPH |
| phenylglyoxal | 19.1 | NADPH |
| 3,4-hexanedione | 2.0 | NADPH |
| 2,3-pentanedione | 7.0 | NADPH |
| ethyl pyruvate | 1.2 | NADPH |
| isoamyl pyruvate | 4.7 | NADPH |
| 2,3-hexanedione | 6.5 | NADPH |
| 1-phenyl-1,2-propanedione | 5.4 | NADPH |
| 2,3-butanedione | 6.0 | NADPH |

| | | |
|---|---|---|
| *U/mg = µmol/mg˙min | | |

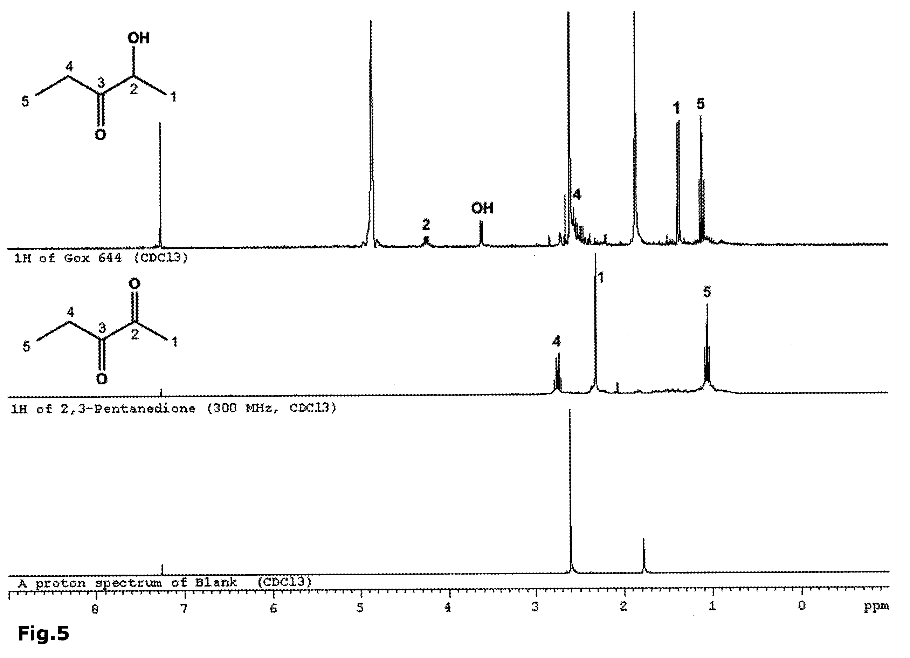

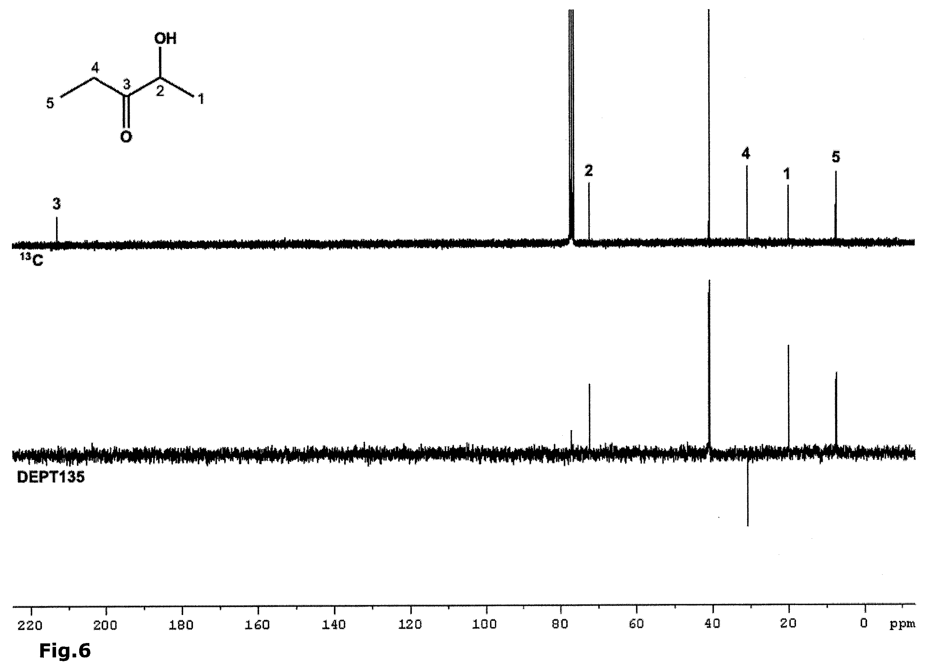

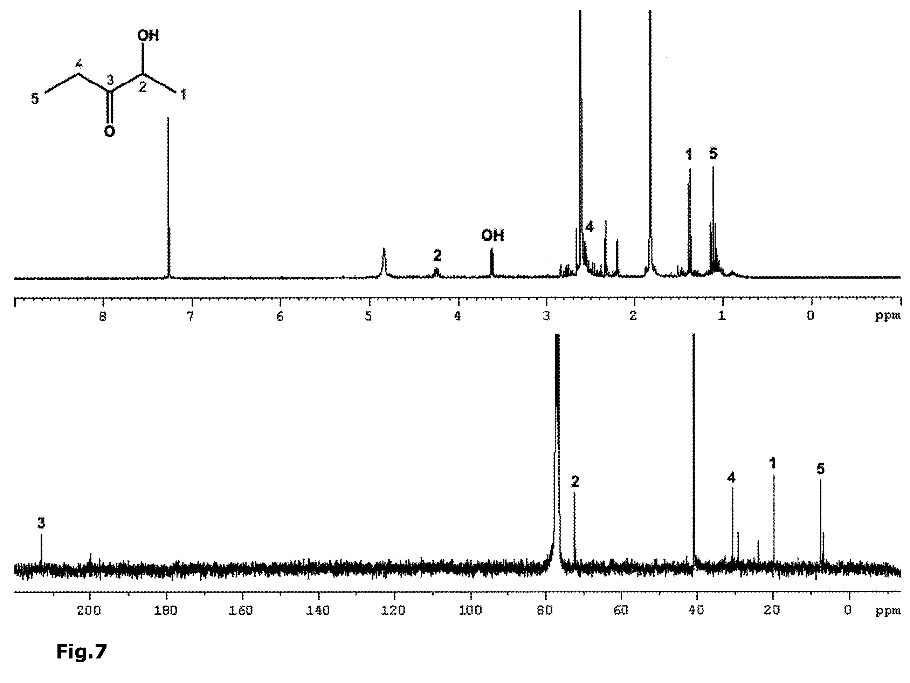

2.7 Regioselectivity of GOX0644 and GOX1615: Tab. 6 displays the regioselective reduction product of Gox0644 and Gox1615 as determined by NMR analysis. The products from the coupled enzyme biotransformations were analyzed by NMR spectrometry (see materials and methods). NMR spectroscopic investigations confirmed the production of 2-hydroxy-pentan-3-one from 2,3-pentanedione for Gox0644 (Fig. 5 and 6) and Gox1615 (Fig. 7). The appearance of a quartett resonance signal at 4.24 ppm and of an exchangeable OH proton at 3.61 ppm in the proton spectrum of Gox0644 indicated the reduction of one ketone group. The shift of the methyl group H₃-1 from δ 2.31 to 1.37 ppm along with the formation of a doublet indicated the reduction of the ketone at position 2. These deductions were further corroborated by analysis of the respective ¹³C, DEPT135 (Fig. 6 and 7) and 2D-NMR spectra. The obtained shift values are also in good agreement with those of synthetically derived 2-hydroxy-pentan-3-one (pechál, M. et al., J. Chromatography 206: 541-547 (1981)).

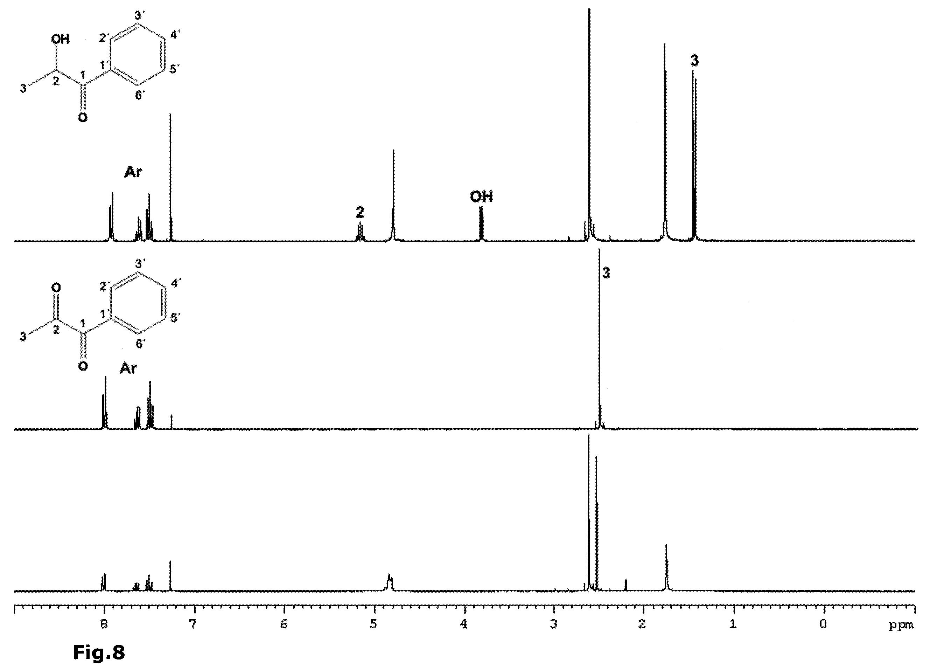

The product of 1-phenyl-1,2-propanedione reduction by Gox0644 was also deduced from ¹H NMR experiments. After biotransformation, the appearance of additional resonances deriving from an oxygen-bearing CH group at δ 5.16, an exchangeable OH proton at 3.82 ppm and a doublet at δ 1.45 (Fig. 8) indicated the reduction of the C-2 keto group, confirming the production of 1-phenyl-2-hydroxy-1-propanone. Commercial standards of 1-phenyl-2-hydroxy-1-propanone and 1-phenyl-1-hydroxy-2-propanone are not available, however the obtained NMR values are in agreement with those of synthetically derived 1-phenyl-2-hydroxy-1-propanone (Toukonitty, E. et al., J. Catal. 204:281-291 (2001); Domínguez de María, P. et al., Adv. Synth. Catal. 350:165-173 (2008)).

**Tab. 6: Summary of Reqioselectivity**

| Enzyme | Substrate | Product |
|---|---|---|
| Gox0644 | 2,3-pentanedione | 2-hydroxy-pentan-3-one |
| Gox0644 | 1-phenyl-1,2-propanedione | 1-phenyl-2-hydroxy-propanone |
| Gox1615 | 2,3-pentanedione | 2-hydroxy-pentan-3-one |

2.8. Stereoselectivity: Tab. 7 displays the stereoselective reduction product of Gox0644 and Gox1615 as determined by NMR analysis. The absolute configuration of the biotransformation product 2-hydroxy-pentan-3-one generated by Gox0644 and Gox1615 have been deduced using a bidentate NMR chiral solvent, as described in Kobayashi, Y. et al., Tetrahedron Lett. 44: 7489-7491 (2003). The negative (-0.004) and positive (+0.010) Δδ*_{RR-SS}*-values of 2-hydroxy-pentan-3-one, produced by Gox0644 were found on the left and right of the secondary carbinol carbon, respectively (Scheme 4), which led to the assignment of the *R* absolute configuration for C-2. In contrast, stereochemical analysis of 2-hydroxy-pentan-3-one, produced by Gox1615 revealed a 2*S* configured product due to positive (+0.007) on the left and negative (-0.014) Δδ*_{RR-SS}*-values on the right of the secondary carbinol carbon (Scheme 4).

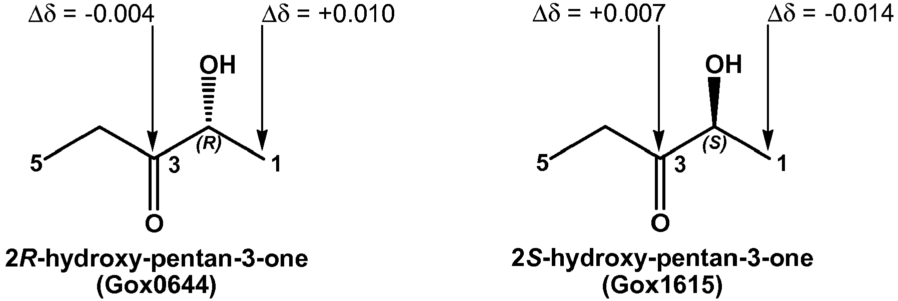

Scheme 4: The behaviour of the ¹³C NMR chemical shifts of the biotransformation product 2-hydroxy-pentan-3-one, produced by Gox0644 and Gox1615 in the chiral NMR solvent BMBA-*p*-Me.

**Table 7: Summary of Stereoselectivity**

| Enzyme | Substrate | Product |
|---|---|---|
| Gox0644 | 2,3-pentanedione | 2*R*-hydroxy-pentan-3-one |
| Gox1615 | 2,3-pentanedione | 2*S*-hydroxy-pentan-3-one |

From said NMR data an ee-value of at least 90% is deducible.

2.9 Kinetic parameters of the keto carbonyl reductases: Kinetic constants were determined from purified enzyme by Lineweaver-Burk plots. The Km and Vmax values for the substrates were calculated using concentrations of 0.025 to 10 mM. The optimal pH values and temperatures for enzyme activities are also shown in Tab. 8.

**Table 8: Summary of kinetic parameters**

| Protein | Substrate | Vmax^{a} (µmol/min x mg) | Km^{a} (mM) | Temp^{b} (°C) | pH optimum |
|---|---|---|---|---|---|
| Gox0644 | 2,3-pentanedione | 69.6 | 5.7 | 35 | 7.0 |
| | Phenylglyoxal | 74.7 | 4.8 | 35 | 7.0 |
| Gox1615 | 2,3-pentanedione | 7.0 | 4.0 | 35 | 6.5 |
| | Methylglyoxal | 23.0 | 4.3 | 35 | 6.5 |
| | | | | | |
| Gox1122 | Acetaldehyde | 196 | 0.2 | 70 | 8.0 |

| | | | | | |
|---|---|---|---|---|---|
| ^{a}Determined with pyrmidine nucletide in 40 mM KPB at optimal pH and 30 °C for each enzyme. ^{b}Temperature optimum | | | | | |

2.10 Bioconversion: 2.5 ml bioconversions were perfomed with 0.1 mg of proteins Gox0644 and Gox1122, 40 mM potassium phosphate buffer, pH 7.0, 50 µM NADPH. Substrates (20 mM 2,3-pentanedione and acetaldehyde) were added every hour. The reaction was followed for 4 h to produce quantities of about 80 mM of product (2-hydroxyl-pentan-3-one; MW = 102 g/mol). Hence, about 20 mg product is formed from 2.5 ml assay mixture. A 1 I *E*. *coli* overproduction culture produces about 40 mg of the pure biocatalyst Gox0644. Therefore, a 1000 ml bioconversion assay can be performed forming 8 g of product in 4 h.

Additionally, 2.5 ml bioconversions were performed with 0.2 mg of proteins Gox1615 and Gox1122, 40 mM potassium phosphate buffer, pH 7.0, 50 µM NADPH. Substrates (20 mM 2,3-pentanedione and acetaldehyde) were added every hour. The reaction was followed for 4 h to produce quantities of about 80 mM of product (2-hydroxyl-pentan-3-one; MW = 102 g/mol). Hence, about 20 mg product is formed from 2.5 ml assay mixture. A 1 I *E. coli* overproduction culture produces about 20 mg of the pure biocatalyst Gox1615. Therefore, a 250 ml bioconversion assays can be performed forming 2 g of product in 4 h.

### Example 3: Description of the Cofactor Regeneration Systems

3.1. Isolation: The *Gluconobacter oxydans* 621H genome contains a gene (*gox*1122) that encodes a putative cytosolic NAD(P)-dependent aldehyde dehydrogenase (isolation and characterization (see Schweiger, P. et al., J. Mol. Microbiol. Biotech. 203:1-9 (2007)). The gene was expressed in *Escherichia coli,* and the recombinant enzyme was purified and characterized. The native protein Gox1122 exhibited an apparent molecular mass of 50.1 kDa, and the subunit mass was 50.5 kDa, indicating a monomeric structure of the native enzyme. The preferred substrates were acetaldehyde and NADP forming acetate and NADPH The enzyme also oxidized other short-chain aliphatic and aromatic aldehydes at lower rates

3.2 Development of the cofactor regeneration system: A prerequisite for the production of α-hydroxycarbonyl compounds is the regeneration of the expensive coenzymes and the stability of the protein catalysts. By coupling two coenzyme-dependent enzyme reactions, NADPH can be continuously regenerated. Hence, instead of stoichiometric amounts, only catalytic amounts of coenzyme are required. For this purpose, a combined enzymatic reaction was used between the NADPH-dependent oxidoreductases that form α-hydroxy carbonyl compounds and the aldehyde dehydrogenase Gox1122 that produces the reduced cofactor in the course of acetaldehyde oxidation. The aldehyde dehydrogenase has very high turnover rates and low Km values for both acetaldehyde and NADP and could be produced in high amounts (Schweiger, P. et al., J. Mol. Microbiol. Biotechnol. 13:147-155 (2007)).

The products derived from the α-diketone and α-ketoaldehyde substrate reactions were generated according to the cofactor regeneration protocol. This method overcomes the dependence of the enzymes on reduced cofactor by coupling the substrate reactions of Gox0644 and Gox1615, with the cofactor NADP reduction reaction based on the acetaldehyde oxidoreductase Gox1122 using acetaldehyde. The results showed that Gox0644 remained very active even at high concentrations (50 mM) of acetaldehyde (Tab. 9). The acetaldehyde tolerance tests showed that at high concentrations (100 mM) the activity of Gox0644 did not show a dramatic decrease. In both the reactions with phenylglyoxal and 2,3-pentanedione, Gox0644 activity remained similar to the control reaction consisting of a reaction without acetaldehyde (Tab. 9/10). Gox0644 with phenylglyoxal and 100 mM of acetaldehyde did not show more than a 20% decrease in activity. In the reaction with 2,3-pentanedione there was a slight decrease of about 20% from the control, however, the activity levels were still very high at 50.2 U/mg. This test demonstrated the ability of Gox0644 to withstand high concentrations of acetaldehyde and allows the use of Gox0644 in a cofactor regeneration reaction in the presence of acetaldehyde.

**Table 9: Gox0644 acetaldehyde tolerance tests with phenylglyoxal**

| Concentration of acetaldehyde | Gox0644 activity* |
|---|---|
| Control | 38.5 U/mg |
| 10mM | 29.6 U/mg |
| 30mM | 37.1 U/mg |
| 50mM | 39.3 U/mg |
| 100mM | 31.2 U/mg |

| | |
|---|---|
| * Gox0644 with phenylglyoxal exhibited high activities in the presence of high concentrations of acetaldehyde; U=µmol/min. | |

**Table 10: Gox0644 acetaldehyde tolerance test with 2,3-pentanedione**

| Concentration of acetaldehyde | Gox0644 activity* |
|---|---|
| Control | 65.7 U/mg |
| 10mM | 67.9 U/mg |
| 30mM | 59.5 U/mg |
| 50mM | 50.1 U/mg |

| | |
|---|---|
| *Gox0644 with 2,3-pentanedione exhibited high activity in the presence of high concentrations of acetaldehyde. This assay demonstrated the high tolerance of the enzyme to acetaldehyde without substantial decrease in activity toward the desired substrate; U=µmol/min | |

3.3. Cofactor regeneration systems: Biotransformations were carried out with Gox1122 oxidizing acetaldehyde to acetate with the concomitant reduction of NADP. This reaction was coupled to NADPH-dependent reductases (e.g. Gox0644 and Gox1615) that reduce carbonyls to recycle the cofactor and allow the addition of cofactor in catalytic amounts instead of stoichiometric amounts. Biotransformation reactions were sampled at various time points and checked for remaining substrate by standard NADP-linked enzyme assays (Schweiger, P. et al., J. Mol. Microbiol. Biotechnol. 13:147-155 (2007)). The amount of remaining substrate was calculated by assuming a 1:1 stoichiometry of cofactor oxidation/reduction to the corresponding substrate oxidation/reduction. The bioconversion reaction was followed for 3 hours to produce quantities between 10-50 mg of product based on the reaction rate.

The cofactor regeneration system for protein Gox644 utilized the oxidative reaction of protein Gox1122 converting acetaldehyde to acetic acid and reducing NADP⁺ to NADPH. The reduced NADPH was oxidized during the reduction of diketones to the hydroxyketone by protein Gox644.

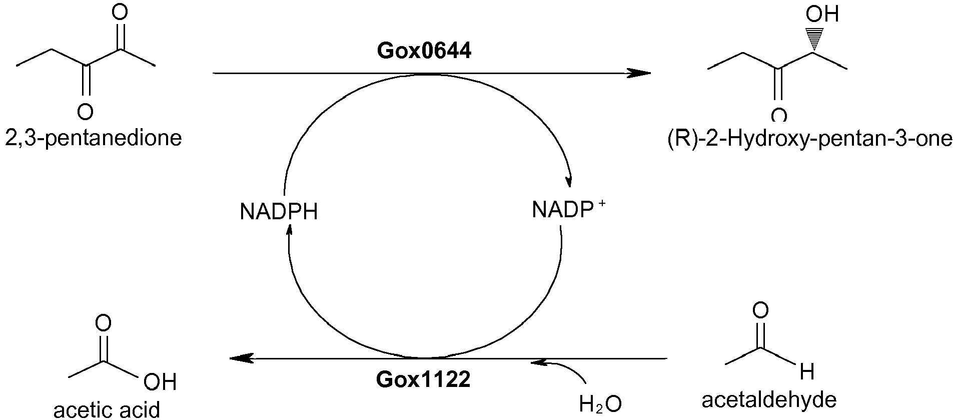

5 ml bioconversions for determining regioselectivity were setup with 2 mg of proteins Gox0644 and Gox1122, 40 mM potassium phosphate buffer, pH 7.0, 50 µM NADPH. Substrates (20 mM 2,3-pentanedione and acetaldehyde) were added every 30 min. The reaction was followed for 3 h to produce quantities between 40-60 mg of product (hydroxy pentanone). NMR product determination showed that Gox0644 specifically reduced the α-ketone group of 2,3-pentanedione to the corresponding 2*R*-hydroxy-pentan-3-one. This conversion resulted in a 100% yield, since no resonances deriving from the educt could be observed in the ¹H and ¹³C NMR spectra after biotransformation. 1-phenyl-1,2-propanedione replaced 2,3-pentanedione in the reaction to produce 1-phenyl-2-hydroxy-propanone, as shown by NMR.

The cofactor regeneration system for protein Gox1615 utilized for the oxidative reaction of protein Gox1122 converting acetaldehyde to acetic acid and reducing NADP⁺ to NADPH. The reduced NADPH was oxidized in the reduction of 2,3-pentanedione to 2*S*-hydroxy-pentan-3-one by protein Gox1615. 5 ml bioconversions for determining regioselectivity were setup with 2 mg of proteins Gox1615 and Gox1122, 40 mM potassium phosphate buffer, pH 7.0, 50 µM NADPH. Substrates (20 mM 2,3-pentandione and acetaldehyde) were added every 30 min. The reaction was followed for 3 h to produce quantities between 40-60 mg of product. NMR confirmed that Gox1615 produced 2*S*-hydroxypentan-3-one. The yield of the biotransformation reaction was 95 % as judged by NMR.

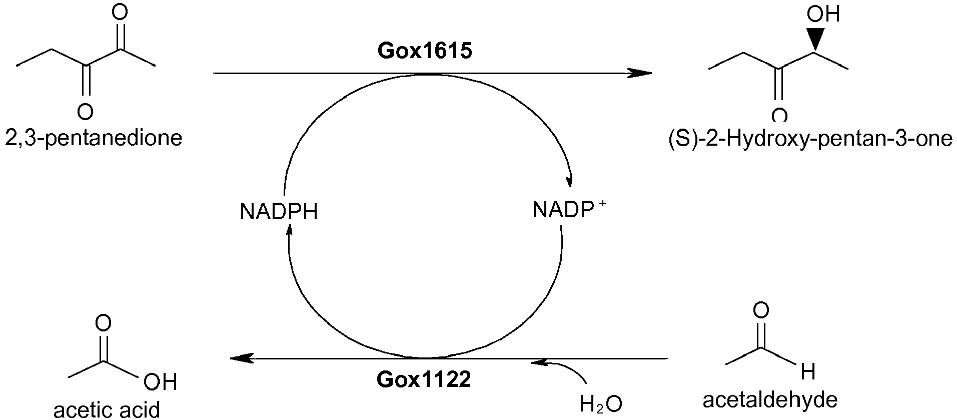

## Claims

1. A process for the enzymatic regio- and enantioselective production of (R)- and (S)-α-hydroxy carbonyl compounds which comprises reacting an α-ketocarbonyl compound with a *Gluconobacter oxydans* α-ketocarbonyl reductase having the amino acid sequence of Gox0644 (SEQ ID NO:2) or Gox1615 (SEQ ID NO:4) or an addition, substitution, inversion and/or deletion mutant thereof having at least 80% sequence identity with the native reductase of SEQ ID NO: 2 or 4, and reducing the α-keto moiety in α-ketoaldehydes and in α-keto esters and the keto moiety of a vicinal diketone that carries the shorter alkyl chain of the molecule, wherein the reduction of the α-ketocarbonyl compound with Gox0644 (SEQ ID NO:2) or the mutant thereof gives an (R)-α-hydroxy carbonyl compound of the structure: 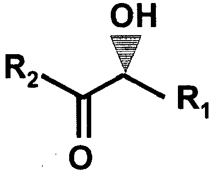 wherein R₁ is alkyl and R₂ is -H, alkyl, aryl, -O-alkyl or -O-aryl, and the reduction of the α-ketocarbonyl compound with Gox1615 (SEQ ID NO:4) or the mutant thereof gives an (S)-α-hydroxy carbonyl compound of the structure: 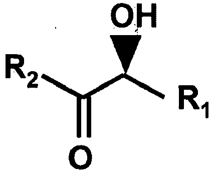 wherein R₁ is alkyl and R₂ is -H, alkyl, aryl, -O-alkyl or -O-aryl.

2. The process of claim 1, wherein the α-ketocarbonyl reductase is Gox0644 having the sequence of SEQ ID NO:2.

3. The process of claim 1 or 2, wherein the (R)-α-hydroxy carbonyl compound is 2*R*-hydroxy-pentan-3-one, 1-phenyl-2*R*-hydroxy-propanone or an (*R*)-α-hydroxy carbonyl compound homologous thereto.

4. The process of claim 1, wherein the α-ketocarbonyl reductase is Gox1615 having the sequence of SEQ ID NO:4.

5. The process of claim 1 or 4, wherein the (S)-α-hydroxy carbonyl compound is 2*S*-hydroxy-pentan-3-one, 1-phenyl-2*S*-hydroxy-propanone or an (*S*)-α-hydroxy carbonyl compound homologous thereto.

6. The process of any one of claims 1 to 5, wherein the reaction is conducted in a cell free environment, wherein the α-ketocarbonyl reductase is present as free enzyme or is presented as a cell lysate or as immobilized enzyme.

7. The process of any one of claims 1 to 5, wherein the reaction is conducted in the presence of a transformant that produces the α-ketocarbonyl reductase *in situ.*

8. The process of claim 7, wherein the transformant or the origin of the cell lysate is a host cell containing at least one of the DNA sequences coding for the α-ketocarbonyl reductase as defined in any one of claims 1 to 3.

9. The process of any one of claims 1 to 8, wherein
(i) the amount of α-ketocarbonyl reductase in the reaction mixture relative to the α-ketocarbonyl compound is in the range of 1 mM to 1 M; and/or
(ii) the reaction is performed in the batch mode or the continuous mode; and/or
(iii) the process further comprises isolating the α-ketocarbonyl reductase from the reaction mixture or the culture broth.

10. The process of any one of claims 1 to 9, wherein the reaction is conducted in the presence of a reducing cofactor, in equimolar or catalytic amount, preferably the reducing cofactor is NADH or NADPH.

11. The process of claim 10, wherein the reducing cofactor NADPH is applied in catalytic amounts and is regenerated with an aldehyde, such as acetaldehyde, and an aldehyde dehydrogenase, preferably the aldehyde dehydrogenase is derived from *Gluconobacter oxydans,* most preferably is Gox1122 (SEQ ID NO:6), or a fragment, derivative or mutant thereof.

## Patentansprüche

1. Verfahren zur enzymatischen regio- und enantioselektiven Herstellung von (R)- und (S)-α-Hydroxycarbonylverbindungen, umfassend
das Umsetzen einer α-Ketocarbonylverbindung mit einer *Gluconobacteroxydans*-α-Ketocarbonyl-Reductase mit der Aminosäuresequenz von Gox0644 (SEQ ID Nr. 2) oder Gox1615 (SEQ ID Nr. 4) oder einer Additions-, Substitutions-, Inversions- und/oder Deletionsmutanten davon, die wenigstens 80% Sequenzidentität mit der nativen Reductase von SEQ ID Nr. 2 oder 4 aufweist, und
das Reduzieren der α-Keto-Struktureinheit in α-Ketoaldehyden und in α-Ketoestern und der Keto-Struktureinheit eines vicinalen Diketons, die die kürzere Alkylkette des Moleküls trägt, wobei die Reduktion der α-Ketocarbonylverbindung mit Gox0644 (SEQ ID Nr. 2) oder dessen Mutante eine (R)-α-Hydroxycarbonylverbindung der Struktur 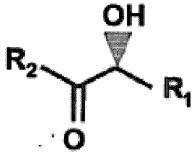 ergibt, wobei R₁ Alkyl ist und R₂ = -H, Alkyl, Aryl, -O-alkyl oder -O-aryl ist und die Reduktion der α-Ketocarbonylverbindung mit Gox1615 (SEQ ID Nr. 4) oder dessen Mutante eine (S)-α-Hydroxycarbonylverbindung der Struktur 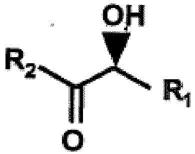 ergibt, wobei R₁ Alkyl ist und R₂ = -H, Alkyl, Aryl, -O-alkyl oder -O-aryl ist.

2. Verfahren gemäß Anspruch 1, wobei es sich bei der α-Ketocarbonyl-Reductase um Gox0644 mit der Sequenz von SEQ ID Nr. 2 handelt.

3. Verfahren gemäß Anspruch 1 oder 2, wobei es sich bei der (R)-α-Hydroxy-carbonylverbindung um 2R-Hydroxypentan-3-on, 1-Phenyl-2R-hydroxy-propanon oder eine dazu homologe (R)-α-Hydroxycarbonylverbindung handelt.

4. Verfahren gemäß Anspruch 1, wobei es sich bei der α-Ketocarbonyl-Reductase um Gox1615 mit der Sequenz von SEQ ID Nr. 4 handelt.

5. Verfahren gemäß Anspruch 1 oder 4, wobei es sich bei der (S)-α-Hydroxy-carbonylverbindung um 2S-Hydroxypentan-3-on, 1-Phenyl-2S-hydroxy-propanon oder eine dazu homologe (S)-α-Hydroxycarbonylverbindung handelt.

6. Verfahren gemäß einem der Ansprüche 1 bis 5, wobei die Reaktion in einer zellfreien Umgebung durchgeführt wird, wobei die α-Ketocarbonyl-Reductase als freies Enzym vorliegt oder als Zelllysat oder immobilisiertes Enzym vorliegt.

7. Verfahren gemäß einem der Ansprüche 1 bis 5, wobei die Reaktion in Gegenwart einer Transformanten durchgeführt wird, die die α-Ketocarbonyl-Reductase in situ produziert.

8. Verfahren gemäß Anspruch 7, wobei die Transformante oder der Ursprung des Zelllysats eine Wirtszelle ist, die wenigstens eine der DNA-Sequenzen enthält, die für die α-Ketocarbonyl-Reductase, wie sie in einem der Ansprüche 1 bis 3 definiert sind, codieren.

9. Verfahren gemäß einem der Ansprüche 1 bis 8, wobei
(i) die Menge der α-Ketocarbonyl-Reductase in dem Reaktionsgemisch relativ zu der α-Ketocarbonylverbindung im Bereich von 1 mM bis 1 M liegt; und/oder
(ii) die Reaktion im diskontinuierlichen Modus oder im kontinuierlichen Modus durchgeführt wird; und/oder
(iii) das Verfahren weiterhin das Isolieren der α-Ketocarbonyl-Reductase aus dem Reaktionsgemisch oder der Kulturbrühe umfasst.

10. Verfahren gemäß einem der Ansprüche 1 bis 9, wobei die Reaktion in Gegenwart eines reduzierenden Cofaktors in äquimolarer oder katalytischer Menge durchgeführt wird, wobei es sich bei dem Cofaktor vorzugsweise um NADH oder NADPH handelt.

11. Verfahren gemäß Anspruch 10, wobei der reduzierende Cofaktor NADPH in katalytischen Mengen angewendet wird und mit einem Aldehyd, wie Acetaldehyd, und einer Aldehyd-Dehydrogenase regeneriert wird, wobei die Aldehyd-Dehydrogenase vorzugsweise von *Gluconobacter oxydans* abgeleitet ist und es sich am meisten bevorzugt um Gox1122 (SEQ ID Nr. 6) oder ein Fragment, Derivat oder eine Mutante davon handelt.

## Revendications

1. Procédé pour la production enzymatique régiosélective et énantiosélective de composés (R)- et (S)-α-hydroxycarbonyle, qui comprend la réaction d'un composé α-cétocarbonyle avec une α-cétocarbonyle-réductase de *Gluconobacter oxydans* ayant la séquence d'acides aminés de Gox0644 (SEQ ID NO : 2) ou de Gox1615 (SEQ ID NO : 4), ou l'un de ses mutants d'addition, de substitution, d'inversion et/ou de délétion, ayant une identité de séquence d'au moins 80% avec la réductase native de SEQ ID NO : 2 ou 4, et la réduction du groupement α-céto en α-cétoaldéhydes et en α-cétoesters et du groupement céto d'une dicétone vicinale qui porte la chaîne alkyle plus courte de la molécule, dans lequel la réduction du composé α-cétocarbonyle avec Gox0644 (SEQ ID NO : 2) ou son mutant donne un composé (R)-α-hydroxycarbonyle de structure : 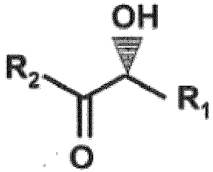 dans laquelle R₁ est un groupe alkyle et R₂ représente -H, un groupe alkyle, aryle, -O-alkyle ou -O-aryle, et la réduction du composé α-cétocarbonyle avec Gox1615 (SEQ ID NO : 4) ou son mutant donne un composé (S)-α-hydroxycarbonyle de structure : 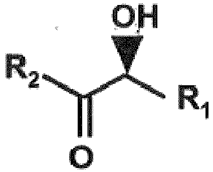 dans laquelle R₁ est un groupe alkyle et R₂ représente -H, un groupe alkyle, aryle, -O-alkyle ou -O-aryle.

2. Procédé selon la revendication 1, dans lequel l'α-cétocarbonyl-réductase est Gox0644 ayant la séquence de SEQ ID NO : 2.

3. Procédé selon la revendication 1 ou 2, dans lequel le composé (R)-α-hydroxycarbonyle est la 2*R*-hydroxy-pentan-3-one, la 1-phényl-2*R-*hydroxy-propanone ou un composé (*R*)-α-hydroxycarbonyle homologue à celles-ci.

4. Procédé selon la revendication 1, dans lequel l'α-cétocarbonyl-réductase est Gox1615 ayant la séquence de SEQ ID NO : 4.

5. Procédé selon la revendication 1 ou 4, dans lequel le composé (S)-α-hydroxycarbonyle est la 2*S*-hydroxy-pentan-3-one, la 1-phényl-2*S*-hydroxy-propanone ou un composé (*S*)-α-hydroxycarbonyle homologue à celles-ci.

6. Procédé selon l'une quelconque des revendications 1 à 5, dans lequel la réaction est effectuée dans un environnement acellulaire, dans lequel l'α-cétocarbonyl-réductase est présente sous forme d'enzyme libre ou est présentée sous forme de lysat cellulaire ou sous forme d'enzyme immobilisée.

7. Procédé selon l'une quelconque des revendications 1 à 5, dans lequel la réaction est effectuée en présence d'un transformant qui produit l'α-cétocarbonyl-réductase in situ.

8. Procédé selon la revendication 7, dans lequel le transformant ou l'origine du lysat cellulaire est une cellule hôte contenant au moins l'une des séquences d'ADN codant pour l'α-cétocarbonyl-réductase telle que définie dans l'une quelconque des revendications 1 à 3.

9. Procédé selon l'une quelconque des revendications 1 à 8, où
(i) la quantité d'α-cétocarbonyl-réductase dans le mélange réactionnel par rapport au composé α-cétocarbonyle est comprise dans l'intervalle de 1 mM à 1 M ; et/ou
(ii) la réaction est effectuée en mode discontinu ou en mode continu ; et/ou
(iii) le procédé comprend en outre l'isolement de l'α-cétocarbonyl-réductase à partir du mélange réactionnel ou du bouillon de culture.

10. Procédé selon l'une quelconque des revendications 1 à 9, dans lequel la réaction est effectuée en présence d'un cofacteur réducteur, en quantité équimolaire ou catalytique, de préférence, le cofacteur réducteur est le NADH ou le NADPH.

11. Procédé selon la revendication 10, dans lequel le cofacteur réducteur NADPH est appliqué en quantités catalytiques et est régénéré à l'aide d'un aldéhyde, tel que l'acétaldéhyde, et d'une aldéhyde-déshydrogénase, de préférence, l'aldéhyde-déshydrogénase est dérivée de *Gluconobacter oxydans*, le plus préférablement, est Gox1122 (SEQ ID NO : 6), ou un de ses fragments, dérivés ou mutants.
